(19) Europäisches Patentamt
European Patent Office
Office européen des brevets

(11) **EP 3 897 517 B1**

(12) **EUROPEAN PATENT SPECIFICATION**

(45) Date of publication and mention
of the grant of the patent:
**28.06.2023  Bulletin 2023/26**

(21) Application number: **18849427.2**

(22) Date of filing: **19.12.2018**

(51) International Patent Classification (IPC):
*A61K 8/06* *(2006.01)*  *A61K 8/19* *(2006.01)*
*A61K 8/29* *(2006.01)*  *A61K 8/31* *(2006.01)*
*A61K 8/36* *(2006.01)*  *A61K 8/37* *(2006.01)*
*A61K 8/49* *(2006.01)*  *A61K 8/81* *(2006.01)*
*A61Q 1/02* *(2006.01)*

(52) Cooperative Patent Classification (CPC):
**A61K 8/062; A61K 8/19; A61K 8/29; A61K 8/31;**
**A61K 8/361; A61K 8/37; A61K 8/375;**
**A61K 8/4993; A61K 8/8158; A61Q 1/02;**
A61K 2800/591; A61K 2800/61

(86) International application number:
**PCT/IB2018/001599**

(87) International publication number:
**WO 2020/128557 (25.06.2020 Gazette 2020/26)**

(54) **OIL-IN-WATER COSMETIC AND METHOD FOR PRODUCING THE SAME**

ÖL-IN-WASSER-KOSMETIK UND VERFAHREN ZU IHRER HERSTELLUNG

COSMÉTIQUE HUILE-DANS-L'EAU ET SON PROCÉDÉ DE FABRICATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**27.10.2021  Bulletin 2021/43**

(73) Proprietor: **L V M H RECHERCHE**
**45800 St. Jean de Braye (FR)**

(72) Inventors:
• **HATA, Koichi**
**Tokyo, 102-0092 (JP)**

• **ASAMI, Yurie**
**Tokyo, 102-0092 (JP)**
• **GOHARA, Hideshi**
**Tokyo, 102-0092 (JP)**
• **SAKODA, Takayoshi**
**Tokyo, 102-0092 (JP)**

(74) Representative: **Regimbeau**
**20, rue de Chazelles**
**75847 Paris Cedex 17 (FR)**

(56) References cited:
**EP-A1- 2 740 774      EP-A1- 2 786 735**
**JP-A- 2017 071 565**

**Description**

**Technical Field**

The present invention relates to an oil-in-water cosmetic and to a method for producing an oil-in-water cosmetic.

**Background Art**

Foundations are cosmetics used to conceal blemishes or fine wrinkles and to improve the aesthetic quality of skin, and because of their major effect on the final appearance of makeup, their level of performance has become a major issue of focus for consumers.

Foundations include cake-type, powder-type, cream-type and liquid-type foundations, among which liquid-type foundations are classified as those with water-in-oil emulsions as bases and those with oil-in-water emulsions as bases (see Japanese Unexamined Patent Publication No. 2008-44901, or its US8728503 equivalent, for example).

Most foundation bases use water-in-oil emulsions (water-in-oil type foundations), because of their high lasting effects and coverage effects for makeup.

The pigments in a water-in-oil type foundation are usually present in a continuous oil phase, so that when they are applied onto skin, the pigments and other components are immediately taken up into the skin, exhibiting a rapid makeup effect.

However, they may also be problematic as they unavoidably produce a dry feeling, since the components of the oil phase are volatile oils used to impart a lighter feel during use, and this results in loss of sebum and moisture of the skin when the foundation is applied.

In recent years, consumers have come to prefer foundations that provide not only makeup effects but also skin care effects, and therefore efforts are being made not only to improve water-in-oil type foundations, but also to develop foundations that use oil-in-water emulsions as bases (oil-in-water foundations).

Oil-in-water foundations, however, which have an aqueous phase as the continuous phase, are highly hydrophilic while having low water resistance and low affinity for the skin, and therefore their makeup ingredients readily drop off from the skin, making it impossible to achieve a lasting effect.

While it has been attempted to provide such foundations with a fresh sensation and a nourishing effect while maintaining an adequate makeup effect, in line with recent consumer preferences, it has not been possible to achieve such types of conflicting functions with prior art technology.

It is therefore an object of this invention to provide an oil-in-water cosmetic that is excellent in terms of providing (1) a fresh sensation, (2) a nourishing effect, (3) a coverage effect and (4) a lasting effect, as well as a method for producing it.

**Summary of the Invention**

The invention provides an oil-in-water cosmetic composed of an aqueous phase and an oil phase, and comprising hydrophobized inorganic powder, a fatty acid soap, a nonionic surfactant and a hydrophilic polymer, wherein the aqueous phase includes an aqueous medium while the oil phase includes an oil containing a non-silicone oil, the hydrophobized inorganic powder includes at least hydrophobized iron oxide powder and hydrophobized pigment-grade titanium oxide powder, with hydrophobized inorganic powder of the same type or different types being present in the oil phase and the aqueous phase, the contents of the hydrophobized pigment-grade titanium oxide powder in the oil phase and in the aqueous phase are, respectively, greater than 0.5 mass% and less than 5 mass%, and greater than 2.5 mass% and less than 20 mass%, based on the total mass of the oil-in-water cosmetic, one or more nonionic surfactants are included, the overall HLB value is from 6 to 15, the hydrophilic polymer contains a hydrophilic polymer including, as a repeating unit, an ethylenic unsaturated compound having an aminoalkylsulfonic acid or salt thereof as a substituent, and the total non-silicone oil content is greater than 5 mass% and less than 30 mass%, based on the total mass of the oil-in-water cosmetic.

It is a feature of the oil-in-water cosmetic of the invention that it has such a structure in which the continuous layer is an aqueous phase and the oil phase is dispersed in it, with hydrophobized inorganic powder of the same type or different types present in both the oil phase and the aqueous phase. Moreover, the contents of specific hydrophobized inorganic powders are within specified ranges, the cosmetic contains a specific oil, nonionic surfactant and hydrophilic polymer, and the content of the oil forming the oil phase is also bounded by an upper limit and a lower limit.

With such oil-in-water cosmetic according to the invention it is possible to exhibit all of the aforementioned effects, including (1) a fresh sensation, (2) a nourishing effect, (3) a coverage effect and (4) a lasting effect. Thus, adequate coverage and lasting effects, that have been difficult to obtain without a water-in-oil cosmetic, are hereby obtainable for the first time with an oil-in-water cosmetic, while the conflicting functions of a fresh sensation and nourishing effect can also be successfully exhibited.

The hydrophobized iron oxide powder and hydrophobized pigment-grade titanium oxide powder in the oil phase and aqueous phase may be hydrophobized by a hydrophobic agent containing one or more selected from the group consisting of trialkoxyalkylsilane, dimethylpolysiloxane, methylhydrosiloxane-dimethylsiloxane copolymer, a salt of amide derived from higher fatty acid and α-amino acid, and alkyl titanate.

Preferably, the hydrophobized iron oxide powder and hydrophobized pigment-grade titanium oxide powder in the oil phase is hydrophobized by a hydrophobic agent containing one or more selected from the group consisting of a salt of amide derived from higher fatty acid and α-amino acid, alkyl titanate, and methylhydrosiloxane-dimethylsiloxane copolymer, and the hydrophobized iron oxide powder and hydrophobized pigment-grade titanium oxide powder in the aqueous phase is hydrophobized by a hydrophobic agent containing one or more selected from the group consisting of dimethylpolysiloxane and methylhydrosiloxane-dimethylsiloxane copolymer.

The properties listed as (1) to (4) above will be more notably exhibited if such hydrophobized inorganic powders are used. If the oil-in-water cosmetic contains only hydrophobized inorganic powders other than those mentioned above, the oil-in-water cosmetic will exhibit poor performance in terms of the (3) coverage effect and (4) lasting effect, and will also fail to exhibit balance between the (1) fresh sensation and (2) nourishing effect.

Ester oils and/or hydrocarbon oils are useful as non-silicone oils. While silicone oils are not excluded for the oil, the oil must include a non-silicone oil, as an essential component. Excellent performance will be exhibited in terms of (1) to (4) above if the non-silicone oil is an ester oil and/or hydrocarbon oil.

The range of applications for the oil-in-water cosmetic is wide and includes foundations, make-up bases, concealers and sunscreens.

The invention also provides a method for producing an oil-in-water cosmetic.

Specifically, it provides a method for producing an oil-in-water cosmetic comprising (A1) a step of combining an oil containing a non-silicone oil, which has been heated, with a dispersion obtained by dispersing a hydrophobized inorganic powder in an oil, which is the same type or a different type from the aforementioned oil, by application of shearing force, and one or more nonionic surfactants having an overall HLB value of 6 to 15, to obtain an oil component in a heated state, (A2) a step of stirring the oil component in a heated aqueous component containing an aqueous medium and a hydrophilic polymer including, as a repeating unit, an ethylenic unsaturated compound having an aminoalkylsulfonic acid or salt thereof as a substituent, to obtain an oil-in-water dispersion, and (A3) a step of cooling the oil-in-water dispersion and adding a hydrophobized inorganic powder, which is the same type or a different type from the aforementioned hydrophobized inorganic powder, to add at least a portion thereof to the aqueous medium, wherein (a1) the oil component and/or aqueous component contains a fatty acid soap, (a2) the hydrophobized inorganic powders in the oil component and aqueous component contain hydrophobized iron oxide powder and hydrophobized pigment-grade titanium oxide powder, the content of the hydrophobized pigment-grade titanium oxide powder in the oil component being greater than 0.5 mass% and less than 5 mass% and the content of the hydrophobized pigment-grade titanium oxide powder in the aqueous component being greater than 2.5 mass% and less than 20 mass%, based on the total mass of the oil-in-water cosmetic, and (a3) the total non-silicone oil content is greater than 5 mass% and less than 30 mass%, based on the total mass of the oil-in-water cosmetic.

The invention further provides a method for producing an oil-in-water cosmetic comprising (B1) a step of combining an oil containing a non-silicone oil, which has been heated, with a dispersion obtained by dispersing a hydrophobized inorganic powder in an oil, which is the same type or a different type from the aforementioned oil, by application of shearing force, one or more nonionic surfactants having an overall HLB value of from 6 to 15, and a fatty acid, to obtain an oil component in a heated state, (B2) a step of stirring the oil component in a heated aqueous component containing an aqueous medium, a basic compound to form a fatty acid soap by pairing with the carboxylic acid anion of the fatty acid, and a hydrophilic polymer including, as a repeating unit, an ethylenic unsaturated compound having an aminoalkylsulfonic acid or salt thereof as a substituent, to obtain an oil-in-water dispersion while forming the fatty acid soap, and (B3) a step of cooling the oil-in-water dispersion and adding a hydrophobized inorganic powder, which is the same type or a different type from the aforementioned hydrophobized inorganic powder, to add at least a portion thereof to the aqueous medium, wherein (b1) the hydrophobized inorganic powders in the oil component and aqueous component contain hydrophobized iron oxide powder and hydrophobized pigment-grade titanium oxide powder, the content of the hydrophobized pigment-grade titanium oxide powder in the oil component being greater than 0.5 mass% and less than 5 mass% and the content of the hydrophobized pigment-grade titanium oxide powder in the aqueous component being greater than 2.5 mass% and less than 20 mass%, based on the total mass of the oil-in-water cosmetic, and (b2) the total non-silicone oil content is greater than 5 mass% and less than 30 mass%, based on the total mass of the oil-in-water cosmetic.

These production methods are the same except for either adding a fatty acid soap (in (a1)), or adding the starting materials for a fatty acid soap (the fatty acid of (B1) and the basic compound of (B2)) and producing a fatty acid soap during production (in step (B2)), and in either case, by carrying out the steps described or using the starting materials described in the timing and amounts mentioned, an oil-in-water cosmetic can be reliably produced that exhibits the performance listed as (1) to (4).

The present invention also relates to a cosmetic process for caring for and/or making-up keratinic materials, comprising the application onto keratinic materials, in particular onto skin, of the oil-in-water cosmetic as defined in the invention.

By 'keratinic materials', it means skin and/or lips, preferably skin.

Advantageously, the oil-in-water cosmetic provides to the keratinoc materials on which it is applied, (1) a fresh sensation, (2) a nourishing effect, (3) a coverage effect and (4) a lasting effect.

**Detailed Description of the Invention**

Preferred embodiments of the invention will now be described, with the understanding that these embodiments are in no way limitative on the invention.

The oil-in-water cosmetic is composed of an aqueous phase and an oil phase, the aqueous phase including an aqueous medium, a hydrophobized inorganic powder and a hydrophilic polymer, and optionally including either or both a fatty acid soap and a nonionic surfactant. The aqueous phase may contain a water-soluble or water-dispersible component in addition to those already mentioned.

The oil phase includes an oil and a hydrophobized inorganic powder, and optionally also includes either or both a fatty acid soap and a nonionic surfactant. The oil phase may additionally contain an oil soluble or dispersible component in addition to those already mentioned.

The oil phase will be explained first.

Oil phase

*Oil(s)*

The oil of the oil phase comprises an oil containing a non-silicone oil, the total non-silicone oil content being greater than 5 mass% and less than 30 mass%, based on the total mass of the oil-in-water cosmetic. A non-silicone oil is an oil lacking a silicone backbone.

If the non-silicone oil content is 5 mass% or less the nourishing effect will be poor, and if it is 30 mass% or greater it will not be possible to obtain a fresh sensation. If only silicone-based oils are included as oils, the fresh sensation, nourishing effect and lasting effect of the oil-in-water cosmetic will all be inferior, and actual formulation of the cosmetic may be difficult.

Non-silicone oils that may be used include hydrocarbon oils, fat oils, waxes, hydrogenated oils, ester oils, fatty acids and higher alcohols (C6 or greater alcohols), from sources such as animal oils, vegetable oils or synthetic oils, regardless of whether they are solid oils, semi-solid oils, liquid oils, volatile oils, and mixtures thereof.

Specific examples include hydrocarbon oils such as liquid paraffins, light liquid isoparaffins, isododecane, isotetradecane, isohexadecane, squalane, vegetable squalane, vaseline, polybutene and hydrogenated polybutene; fat oils such as olive oil, castor oil, macadamia nut oil, camellia oil, rose hip oil and avocado oil; ester oils such as jojoba oil, cetyl 2-ethylhexanoate, isononyl isononanoate, isotridecyl isononanoate, isopropyl myristate, isopropyl palmitate, octyldodecyl myristate, isotridecyl isononanoate, glyceryl tri(2-ethylhexanoate), propyleneglycol dicaprate, neopentyl glycol dicaprate, diglyceryl triisostearate, glyceryl tribehenate, diisostearyl malate and ethylhexyl methoxycinnamate; and lanolin or lanolin derivatives such as lanolin acetate and lanolin alcohol, and mixtures thereof . Any of which may be used alone or in combinations of two or more.

Polar oils are preferred, with fat oils, ester oils and lanolin derivatives being preferred polar oils. Particularly preferred fat oils include olive oil, castor oil, macadamia nut oil, camellia oil, rose hip oil, avocado oil and mixtures thereof. Particularly preferred ester oils include cetyl 2-ethylhexanoate, isononyl isononanoate, isotridecyl isononanoate, isopropyl myristate, isopropyl palmitate, octyldodecyl myristate, isotridecyl isononanoate, glyceryl tri(2-ethylhexanoate), propyleneglycol dicaprate, diisostearyl malate, ethylhexyl methoxycinnamate and mixtures thereof. Particularly preferred lanolin derivatives include lanolin acetate and lanolin alcohol, while ethylhexyl methoxycinnamate is particularly preferred as a polar oil with an ultraviolet absorption effect.

The non-silicone oil content may be a minimum of 6 mass%, 7 mass% or 10 mass%, to a maximum of 28 mass%, 25 mass% or 20 mass%, based on the total mass of the oil-in-water cosmetic. That is, the non-silicone oil content may be from 6 to 28 mass%, from 6 to 25 mass%, from 6 to 20 mass%, from 7 to 28 mass%, from 7 to 25 mass%, from 7 to 20 mass%, from 10 to 28 mass%, from 10 to 25 mass% or preferably from 10 to 20 mass%, based on the total mass (weight) of the oil-in-water cosmetic.

*Hydrophobized inorganic powder*

The oil phase includes a hydrophobized inorganic powder, which may be hydrophobized iron oxide powder and hydrophobized pigment-grade titanium oxide powder. The content of hydrophobized pigment-grade titanium oxide powder in the oil phase is greater than 0.5 mass% and less than 5 mass%, based on the total mass of the oil-in-water cosmetic.

"Hydrophobized" means that inorganic powder is treated with a hydrophobic agent to modify the surface of the untreated inorganic powder for increased hydrophobicity, as explained in detail below. Titanium oxide powder is largely classified as pigment-grade titanium oxide powder, having a mean particle size of 200 nm or greater, or fine particulate titanium oxide powder, having a mean particle size of less than 200 nm (typically 100 nm or smaller), and the oil-in-water cosmetic must contain at least pigment-grade titanium oxide powder. As long as the oil-in-water cosmetic contains pigment-grade titanium oxide powder it may also contain fine particulate titanium oxide. The mean particle size of the hydrophobized pigment-grade titanium oxide powder is preferably 200 to 400 nm, and the mean particle size of the hydrophobized iron oxide powder is preferably 100 to 500 nm.

If the content of the hydrophobized pigment-grade titanium oxide powder in the oil phase is 0.5 mass% or less based on the total mass of the oil-in-water cosmetic, the (3) coverage effect and (4) lasting effect will be poor, while if it is 5 mass% or greater, the (1) fresh sensation and (2) nourishing effect will be poor and actual formulation of the cosmetic may be difficult.

The hydrophobic agent used to hydrophobize the iron oxide powder and pigment-grade titanium oxide powder may contain silane, silicone, a fluorine-based compound, an organic titanate compound, an amide or its salt, a metal soap, hydrocarbon, higher fatty acid, higher alcohol, ester or wax. These hydrophobic agents may also be used in combination.

Trialkoxy alkylsilanes are common silanes, and dimethylpolysiloxane (also known as dimethicone) and methylhydrosiloxane-dimethylsiloxane copolymer (also known as (dimethicone/methicone) copolymer or methylhydrogenpolysiloxane) are common silicones. Organic titanates include alkyl titanate, and amide salts include a salt of amide derived from higher fatty acid and $\alpha$-amino acid.

Trialkoxyalkylsilanes are compounds having three alkoxy groups and one alkyl group bonded to a silicon atom, and the alkoxy groups react with the hydroxyl groups or other reactive group(s) on the powder surfaces to cover them. The alkoxy groups of a trialkoxyalkylsilane are preferably C1-3 alkoxy groups such as methoxy, ethoxy or propoxy. The alkyl group of a trialkoxyalkylsilane is preferably a C6-18 alkyl group such as hexyl, octyl, decyl or octadecyl.

Examples of such trialkoxyalkylsilanes include trimethoxyhexylsilane, trimethoxyoctylsilane, trimethoxydecylsilane, trimethoxyoctadecylsilane, triethoxyhexylsilane, triethoxyoctylsilane, triethoxydecylsilane and triethoxyoctadecylsilane,with triethoxyoctylsilane being particularly preferred for satisfactory dispersibility in the oil phase. One trialkoxyalkylsilane or two or more in combination may be used.

Alkyl titanate may be a long-chain carboxylic acid-type, pyrophosphoric acid-type, phosphorous acid-type or amino acid-type, but from the viewpoint of dispersion stability it is preferably alkyl titanate with a C8-24 alkyl group, examples of such compounds including those represented by the following formula (I):

$$(R^1O)\text{-}Ti\text{-}(OCOR^2)_3 \qquad (I)$$

(wherein $R^1$ is a C1-4 alkyl group and $R^2$ is a C8-24 alkyl group, both of which may be either straight-chain or branched).

Alkyl titanates include long-chain carboxylic acid-type alkyl titanates such as isopropyltriisostearoyl titanate, isopropyltrioctanoyl titanate, isopropyldimethacrylisostearoyl titanate, isopropylisostearoyldiacryl titanate and diisostearoylethylene titanate, pyrophosphoric acid-type alkyl titanates such as tetraisopropylbis(dioctyl phosphite) titanate, tetraoctylbis(ditridecyl phosphite) titanate and tetra(2,2-diallyloxymethyl-1-butyl)bis(ditridecyl phosphite) titanate, phosphorous acid-type alkyl titanates such as isopropyl tri(dioctylpyrophosphate)titanate and bis(dioctylpyrophosphate)ethylene titanate, and amino acid-type alkyl titanates such as isopropyltri(N-amideethyl-aminoethyl) titanate, any of which may be used alone or in combinations of two or more, although using isopropyltriisostearoyl titanate is preferred among these alkyl titanates as it exhibits excellent dispersibility in oil phases.

A salt of amide derived from higher fatty acid and $\alpha$-amino acid is preferably a metal salt of amide derived from higher fatty acid and $\alpha$-amino acid (especially an alkyl metal salt such as a sodium salt or potassium salt, or an alkyl earth metal salt).

A higher fatty acid means a C12 or greater fatty acid, examples of which include dodecanoic acid (lauric acid), tetradecanoic acid (myristic acid), pentadecanoic acid, hexadecanoic acid (palmitic acid), 9-hexadecenoic acid, heptadecanoic acid, octadecanoic acid (stearic acid), cis-9-octadecenoic acid, 11-octadecenoic acid, cis,cis-9,12-octadecadienoic acid, 9,12,15-octadecatrienoic acid, 6,9,12-octadecatrienoic acid, 9,11,13-octadecatrienoic acid, eicosanoic acid, 8,11-eicosadienoic acid, 5,8,11-eicosatrienoic acid, 5,8,11-eicosatetraenoic acid, docosanoic acid, tetracosanoic acid, cis-15-tetracosanoic acid, hexacosanoic acid, octacosanoic acid and triacontanoic acid.

Examples of α-amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine.

Dimethylpolysiloxane may be used as a surface treatment agent to impart hydrophobicity to the powder, in addition to its usual purpose as an oil. Methylhydrosiloxane-dimethylsiloxane copolymer is a copolymer of dimethylsiloxane and methylhydrogensiloxane, which can be used to impart hydrophobicity to a powder while functioning as a surface treatment agent having low hydrogen generation with time.

The hydrophobized iron oxide powder and hydrophobized pigment-grade titanium oxide powder in the oil phase are preferably made by hydrophobizing non-hydrophobized powders with a hydrophobic agent including either or both alkyl titanate and a salt of amide derived from higher fatty acid and α-amino acid, and more preferably they are made with a hydrophobic agent including both alkyl titanate and a salt of amide derived from higher fatty acid and α-amino acid. The alkyl titanate is preferably isopropyltitanium triisostearate, and the salt of amide derived from higher fatty acid and α-amino acid is preferably sodium lauroyl aspartate.

The degree of hydrophobic treatment of the hydrophobized inorganic powder to be added to the oil phase may be a minimum of 0.5 mass%, 1% mass% or 2 mass%, up to a maximum of 15% mass%, 12 mass% or 10 mass%, based on the mass of the inorganic powder after it is hydrophobized. In other words, the degree of hydrophobic treatment of the hydrophobized inorganic powder may be from 0.5 to 15 mass%, from 0.5 to 12 mass%, from 0.5 to 10 mass%, from 1 to 15 mass%, from 1 to 12 mass%, from 1 to 10 mass%, from 2 to 15 mass%, from 2 to 12 mass% or preferably from 2 to 10 mass%.

A mixture of several hydrophobized iron oxide powders may also be used as a component of the hydrophobized inorganic powder in the oil phase. For example, it may be a mixture of hydrophobized iron oxide powder with a black appearance (black hydrophobized iron oxide powder), hydrophobized iron oxide powder with a red appearance (red hydrophobized iron oxide powder) and hydrophobized iron oxide powder with a yellow appearance (yellow hydrophobized iron oxide powder), with adjustment to a color tone to match the skin onto which it is to be applied.

The contents of black hydrophobized iron oxide powder, red hydrophobized iron oxide powder and yellow hydrophobized iron oxide powder may each be a minimum of 0.2 mass%, 0.3 mass% or 0.4 mass%, up to a maximum of 4 mass%, 3.5 mass% or 3 mass%, based on the total mass of the oil-in-water cosmetic. In other words, the powder contents may each be from 0.2 to 4 mass%, from 0.2 to 3.5 mass%, from 0.2 to 3 mass%, from 0.3 to 4 mass%, from 0.3 to 3.5 mass%, from 0.3 to 3 mass%, from 0.4 to 4 mass%, from 0.4 to 3.5 mass% or preferably from 0.4 to 3 mass%, based on the total mass of the oil-in-water cosmetic.

The content of the hydrophobized pigment-grade titanium oxide powder as a component of the hydrophobized inorganic powder in the oil phase may be a minimum of 0.75 mass%, 1 mass% or 1.25 mass%, up to a maximum of 4.75 mass%, 4.25 mass% or 3.75 mass%, based on the total mass of the oil-in-water cosmetic. In other words, the contents of the hydrophobized pigment-grade titanium oxide powders may each be from 0.75 to 4.75 mass%, from 0.75 to 4.25 mass%, from 0.75 to 3.75 mass%, from 1 to 4.75 mass%, from 1 to 4.25 mass%, from 1 to 3.75 mass%, from 1.25 to 4.75 mass%, from 1.25 to 4.25 mass% or preferably from 1.25 to 3.75 mass%, based on the total mass of the oil-in-water cosmetic. The content of the pigment-grade titanium oxide powder itself, subtracting the hydrophobized weight from the hydrophobized pigment-grade titanium oxide powder, may be greater than 0.47 mass% and less than 4.7 mass%, based on the total mass of the oil-in-water cosmetic.

*Fatty acid soaps*

The oil phase may include one or more fatty acid soaps. Fatty acid soaps are formed from "a fatty acid" and "a basic compound to form a fatty acid soap by pairing with the carboxylic acid anion of the fatty acid" (hereunder also referred to simply as "basic compound"), and they may be added to the aqueous phase and/or oil phase of the oil-in-water cosmetic with the fatty acid and the basic compound as a pair (an example of "pair" : salt), or the fatty acid and basic compound may be each added respectively to the starting material that is to form the oil phase (oil component) and the starting material that is to form the aqueous phase (aqueous component), so that a fatty acid soap is formed during final formulation of the oil-in-water cosmetic. The fatty acid soap may also be present at the interface between the oil phase and the aqueous phase.

The fatty acid of the fatty acid soap is preferably lauric acid, myristic acid, palmitic acid, stearic acid, behenic acid, or mixtures thereof, since a solid at room temperature (25°C) is preferred.

The basic compound of the fatty acid soap, on the other hand, only needs to be one that forms, for example, a salt with the fatty acid, and suitable examples include metal hydroxides such as sodium hydroxide and potassium hydroxide, and amines such as 2-amino-2-methyl-1,3-propanediol, tromethamine (2-amino-2-hydroxymethyl-1,3-propanediol), triethanolamine and aminomethylpropanol.

Fatty acid soaps are thus classified in general as fatty acid metal salts or fatty acid ammonium salts.

Fatty acid metal salts include potassium laurate, coconut fatty acid potassium, potassium myristate, potassium palmitate, potassium stearate, potassium behenate, potassium oleate, sodium laurate, coconut fatty acid sodium, sodium myristate, sodium palmitate, sodium stearate, sodium behenate and sodium oleate.

Fatty acid ammonium salts include compounds formed from one or more selected from the group consisting of lauric acid, myristic acid, palmitic acid, stearic acid and behenic acid, and one or more selected from the group consisting of 2-amino-2-methyl-1,3-propanediol, tromethamine (2-amino-2-hydroxymethyl-1,3-propanediol), triethanolamine and aminomethylpropanol.

The total content of the fatty acid soap, or the fatty acid as the fatty acid soap precursor, may be a minimum of 0.1 mass%, 0.2 mass% or 0.3 mass%, up to a maximum of 3 mass%, 2 mass% or 1 mass%, based on the total mass of the oil-in-water cosmetic. In other words, the fatty acid soap content may be from 0.1 to 3 mass%, from 0.1 to 2 mass%, from 0.1 to 1 mass%, from 0.2 to 3 mass%, from 0.2 to 2 mass%, from 0.2 to 1 mass%, from 0.3 to 3 mass%, from 0.3 to 2 mass% or preferably from 0.3 to 1 mass%, based on the total mass of the oil-in-water cosmetic.

*Nonionic surfactants*

The oil phase may also include one or more nonionic surfactants. The nonionic surfactant will usually be present in the oil phase, but it may be present in either or both the oil phase and aqueous phase, or at the interface between the oil phase and the aqueous phase.

The HLB (Hydrophile-Lipophile Balance) value of the nonionic surfactant as a whole is in the range of 6 to 15, the value being preferably from 7 to 12 and more preferably from 8 to 10.

The HLB value may be measured by the Griffin method, or it may be calculated from the Inorganic-Organic Balance (IOB) value, obtained by an organic paradigm method (for example, Koda, Y., "Yuki Gainenzu - Kiso to Ouyou", pp.11-17, Sankyo Publishing, 1984), and it is expressed as follows.

$$\text{HLB value} = \text{IOB value} \times 10$$

When the nonionic surfactant is a mixture of two or more components, the HLB value is defined as the weighted average of the HLB value for each component.

A nonionic surfactant having an overall HLB value of 6 to 15 can be obtained by combining a nonionic surfactant having an HLB value of 3 to 9 (hereunder also referred to as "low HLB nonionic surfactant") and a nonionic surfactant having an HLB value of 10 to 18 (hereunder also referred to as "high HLB nonionic surfactant"), for example.

The low HLB nonionic surfactant used may be a glycerin fatty acid ester, polyglyceryl-type surfactant, sorbitan fatty acid ester, polyhydroxystearic acid or sucrose fatty acid ester, with any one or more of these selected as necessary. Polyglyceryl-type surfactants, sorbitan fatty acid esters and polyhydroxystearic acid are preferred for higher dispersibility and more excellent stability in the oil phase.

A polyglyceryl-type surfactant that is used preferably has a glycerin polymerization degree of 2 to 4, such polyglyceryl-type surfactants including diglyceryl isostearate, polyglyceryl isostearate, diglyceryl diisostearate and polyglyceryl diisostearate. Commercial products are available, including COSMOL 41V (Nisshin OilliO Group, Ltd.) as polyglyceryl isostearate, and COSMOL 42V (Nisshin OilliO Group, Ltd.) as polyglyceryl diisostearate.

Sorbitan fatty acid esters include sorbitan oleate, sorbitan isostearate and sorbitan sesquioleate. Commercial products are available, including COSMOL 182V (Nisshin OilliO Group, Ltd.) as sorbitan oleate, and SPAN83 (Croda Co.) and RHEODOL AO-15 (Kao Corp.) as sorbitan sesquioleate. Other commercial products include SPAN80 (HLB value: 4.3) and SPAN60 (HLB value: 4.7) by Croda Co.

Polyhydroxy stearic acid that is used preferably has a hydroxyl group at position 12, with a hydroxystearic acid polymerization degree of preferably 3 to 12 and more preferably 4 to 8. ARLACEL P-100 (Unichema Co.) is an example of a commercially available product.

The high HLB nonionic surfactant may be a polyoxyalkylene alkyl ether, polyoxyalkylene fatty acid ester, polyoxyethylene hydrogenated castor oil, polyoxyethylene sorbitan fatty acid ester, polyoxyethylene glycerin fatty acid ester or polyoxyalkylene-modified silicone, for example, any of which may be used alone or in combinations of two or more, as necessary.

Commercial products that are available include the polyoxyethylene sorbitan fatty acid esters: TWEEN65 (HLB value: 10.5), TWEEN21 (HLB value: 13.3), TWEEN60 (HLB value: 14.9), TWEEN80 (HLB value: 15.0), TWEEN40 (HLB value: 15.6) and TWEEN20 (HLB value: 16.7), by Croda Co.

When an HLB value of 6 to 15 is desired using a single nonionic surfactant, the sorbitan fatty acid esters SPAN40 (HLB value: 6.7) or SPAN20 (HLB value: 8.6) by Croda Co., or the polyoxyethylene sorbitan fatty acid esters TWEEN65 (HLB value: 10.5), TWEEN21 (HLB value: 13.3), TWEEN60 (HLB value: 14.9) or TWEEN80 (HLB value: 15.0), also by Croda Co, may be used.

The total nonionic surfactant content may be a minimum of 0.1 mass%, 0.25 mass% or 0.5 mass%, to a maximum of 5 mass%, 3 mass% or 2 mass%, based on the total mass of the oil-in-water cosmetic. In other words, the nonionic surfactant content may be from 0.1 to 5 mass%, from 0.1 to 3 mass%, from 0.1 to 2 mass%, from 0.25 to 5 mass%, from 0.25 to 3 mass%, from 0.25 to 2 mass%, from 0.5 to 5 mass%, from 0.5 to 3 mass% or preferably from 0.5 to 2 mass%, based on the total mass of the oil-in-water cosmetic.

*Oil soluble or dispersible component*

The oil phase may additionally contain an oil soluble or dispersible component in addition to the components already mentioned. Such additional components may be emulsification aids, dispersing agents, oil-soluble ultraviolet absorbers or oil-soluble vitamins.

An emulsification aid will usually be present in the oil phase, but it may be present in either or both the oil phase and aqueous phase, or at the interface between the oil phase and the aqueous phase.

Examples of emulsification aids that may be used include C 12-24 straight-chain saturated alcohols and monoglycerin fatty acid esters.

C12-24 straight-chain saturated alcohols include myristyl alcohol, hexadecyl alcohol, cetyl alcohol, cetostearyl alcohol, stearyl alcohol, arachidyl alcohol and behenyl alcohol, among which any one or combination of two or more may be used. Cetostearyl alcohol, stearyl alcohol or behenyl alcohol may be selected to obtain an oil-in-water emulsified composition having very satisfactory stability over time, with stearyl alcohol being especially preferred.

A monoglycerin fatty acid ester may be lipophilic or self-emulsifiable, and the fatty acid of the monoglycerin fatty acid ester may be any of those mentioned above as precursors for the fatty acid soap.

Monoglycerin fatty acid esters include glyceryl monostearate, which is commercially available as Nikkol MGS-ASEV (Nikko Chemicals Co.) or POEM V-100 (Riken Vitamin Co., Ltd.).

The total emulsification aid content may be a minimum of 0.1 mass%, 0.25 mass% or 0.5 mass% to a maximum of 5 mass%, 3 mass% or 2 mass%, based on the total mass of the oil-in-water cosmetic. In other words, the emulsification aid content may be from 0.1 to 5 mass%, from 0.1 to 3 mass%, from 0.1 to 2 mass%, from 0.25 to 5 mass%, from 0.25 to 3%, from 0.25 to 2 mass%, from 0.5 to 5 mass%, from 0.5 to 3 mass% or preferably from 0.5 to 2 mass%, based on the total mass of the oil-in-water cosmetic.

The oil phase may also include a dispersing agent. A dispersing agent may be added to the non-silicone oil during production of the oil-in-water cosmetic when shearing force is to be applied to the non-silicone oil to obtain a dispersion of the hydrophobized inorganic powder. The dispersing agent will usually be present in the oil phase, but it may be present in either or both the oil phase and aqueous phase, or at the interface between the oil phase and the aqueous phase.

The type of dispersing agent used may be the same as the nonionic surfactant mentioned above. From the viewpoint of dispersibility in the oil phase, however, it is preferably a low HLB nonionic surfactant (a nonionic surfactant with an HLB value of 3 to 5).

Specifically, the dispersing agent used may be a glycerin fatty acid ester, polyglyceryl-type surfactant, sorbitan fatty acid ester, polyhydroxystearic acid or sucrose fatty acid ester, with any one or more of these selected as necessary. Specific examples and their preferred cases are the same as above.

The total dispersing agent content may be a minimum of 0.01 mass%, 0.03 mass% or 0.05 mass% up to a maximum of 1 mass%, 0.5 mass% or 0.2 mass%, based on the total mass of the oil-in-water cosmetic. In other words, the emulsification aid content may be from 0.01 to 1 mass%, from 0.01 to 0.5 mass%, from 0.01 to 0.2 mass%, from 0.03 to 1 mass%, from 0.03 to 0.5 mass%, from 0.03 to 0.2 mass%, from 0.05 to 1 mass%, from 0.05 to 0.5 mass% or preferably from 0.05 to 0.2 mass%, based on the total mass of the oil-in-water cosmetic.

Examples of oil-soluble ultraviolet absorbers to be added in the oil phase include benzoic acid-based ultraviolet absorbers such as paraaminobenzoic acid (PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester and hexyl diethylaminohydroxybenzoylbenzoate; anthranilic acid-based ultraviolet absorbers such as homomenthyl-N-acetyl anthranilate; salicylic acid-based ultraviolet absorbers such as amyl salicylate, menthyl salicylate, homomenthyl salicylate, octyl salicylate, phenyl salicylate, benzyl salicylate, p-isopropanolphenyl salicylate; cinnamic acid-based ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate, 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate, glycerylmono-2-ethylhexanoyl-diparamethoxy cinnamate and 3-methyl-4-[methylbis(trimethylsiloxy)silyl]butyl 3,4,5-trimethoxycinnamate; and 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenylbenzotriazole, dibenzalazine, dianisoylmethane, 4-tert-butyl-4'-methoxydibenzoylmethane, 4-(1,1-dimethylethyl)-4'-methoxydibenzoylmethane, 2-cyano-3,3-diphenyl-2-propenoic acid-2-ethylhexyl ester dimethicone-diethylbenzal malonate, and mixtures thereof.

The aqueous phase will now be described.

Aqueous Phase

As mentioned above, the aqueous phase includes an aqueous medium, a hydrophobized inorganic powder and a hydrophilic polymer, and optionally also includes either or both a fatty acid soap and a nonionic surfactant. The aqueous phase may contain a water-soluble or water-dispersible component in addition to those already mentioned.

*Aqueous medium*

The aqueous medium comprises water as an essential component. The water used may be distilled water, purified water, hot spring water, deep water, or plant-derived steam distilled water such as lavender water, rose water or orange flower water.

The water content may be a minimum of 20 mass%, 25 mass% or 30 mass%, up to a maximum of 80 mass%, 70 mass% or 60 mass%, based on the total mass of the oil-in-water cosmetic. In other words, the water content may be from 20 to 80 mass%, from 20 to 70 mass%, from 20 to 60 mass%, from 25 to 80 mass%, from 25 to 70 mass%, from 25 to 60 mass%, from 30 to 80 mass%, from 30 to 70 mass% or preferably from 30 to 60 mass%, based on the total mass of the oil-in-water cosmetic.

Water-soluble or water-dispersible components that may be added to the aqueous phase include preservatives, low molecular mono-alcohols (C1-C6 mono-alcohols), water-soluble ultraviolet absorbers, antimicrobial agents, antiinflammatory drugs, water-soluble vitamins and amino acids. The aqueous phase may also include an emulsification aid or dispersing agent in addition to either or both a fatty acid soap and a nonionic surfactant.

Aryloxyalkanols such as phenoxyethanol are useful as preservatives, while ethanol and propanol may be used as low molecular mono-alcohols.

Examples of water-soluble ultraviolet absorbers include benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid salt, 4-phenylbenzophenone, 2-ethylhexyl-4'-phenyl-benzophenone-2-carboxylate, 2-hydroxy-4-n-octoxybenzophenone and 4-hydroxy-3-carboxybenzophenone; benzimidazole-based ultraviolet absorbers such as phenylbenzimidazole-5-sulfonic acid and its salts and phenylene-bis-benzimidazole-tetrasulfonic acid and its salts; benzotriazole-based ultraviolet absorbers such as methylene bis-benzotriazolyl-tetramethylbutylphenol; and 3-(4'-methylbenzylidene)-d,l-camphor, 3-benzylidene-d,l-camphor, urocanic acid, ethyl urocanate ester, and mixtures thereof.

*Hydrophilic polymer*

The aqueous phase contains a hydrophilic polymer, the hydrophilic polymer itself containing, as an essential component, a hydrophilic polymer including, as a repeating unit, an ethylenic unsaturated compound having an aminoalkylsulfonic acid or its salt as a substituent (hereunder also referred to as "aminoalkylsulfonic acid polymer"). Hydrophilic polymers include, in addition to the water-soluble polymers, also polymers that swell with water or that are microdispersed in water.

Useful aminoalkylsulfonic acid polymers include (meth)acrylic polymers having, as repeating units, N-vinylpyrrolidone and a (meth)acrylamide having an aminoalkylsulfonic acid or its salt as a substituent. The term "(meth)acrylic" refers to acrylic or methacrylic, and the same applies to analogous compounds.

The aforementioned substituents include alkyl-substituted aminoalkylsulfonic acid groups such as alkyl-substituted taurine groups, which in turn include dimethyltaurine ($-NH-C(CH_3)_2-CH_2-SO_3H$). Such a hydrophilic polymer is commercially available as (acryloyldimethyltaurineammonium/VP) copolymer (trade name: ARISTOFLEX AVC, by Clariant Japan), for example.

Hydrophilic polymers that may be used together with aminoalkylsulfonic acid polymers include hydrophilic polymers that have (meth)acrylic acid as a repeating unit (hereunder also referred to as "acrylic acid polymers"). Acrylic acid polymers may be either homopolymers or copolymers with other ethylenic unsaturated compounds, and may be either non-crosslinking types or crosslinkable types. Crosslinkable acrylic acid polymers include carboxyl group-containing polymers, with allylsucrose or pentaerythritol as crosslinking agents. CARBOPOL 980 (by Lubrizol Co.) is one commercially available hydrophilic polymer.

An acrylic acid polymer can be used in combination with an aminoalkylsulfonic acid polymer, but if an acrylic acid polymer is to be used alone without an aminoalkylsulfonic acid polymer, the (1) fresh sensation, (2) nourishing effect, (3) coverage effect and (4) lasting effect are impaired. Thus, selection of the hydrophilic polymer is important. The hydrophilic polymer is preferably an aminoalkylsulfonic acid polymer, used alone.

The hydrophilic polymer content may be a minimum of 0.3 mass%, 0.5 mass% or 0.8 mass%, up to a maximum of 5 mass%, 2.5 mass% or 2 mass%, based on the total mass of the oil-in-water cosmetic. In other words, the hydrophilic polymer content may be from 0.3 to 5 mass%, from 0.3 to 2.5 mass%, from 0.3 to 2 mass%, from 0.5 to 5 mass%, from 0.5 to 2.5 mass%, from 0.5 to 2 mass%, from 0.8 to 5 mass%, from 0.8 to 2.5 mass% or preferably from 0.8 to 2 mass%, based on the total mass of the oil-in-water cosmetic.

*Hydrophobized inorganic powder*

The aqueous phase includes a hydrophobized inorganic powder of the same type or a different type as the one in the oil phase, and it may be hydrophobized iron oxide powder and hydrophobized pigment-grade titanium oxide powder. The content of hydrophobized pigment-grade titanium oxide powder in the aqueous phase is greater than 2.5 mass% and less than 20 mass%, based on the total mass of the oil-in-water cosmetic.

If the content of the hydrophobized pigment-grade titanium oxide powder in the aqueous phase is 2.5 mass% or less, the (3) coverage effect and (4) lasting effect will be poor, while if it is 20 mass% or greater, the (1) fresh sensation and (2) nourishing effect will be inadequate.

Hydrophobizing for the hydrophobized inorganic powder in the aqueous phase can be carried out using the same type of hydrophobic agent as used for hydrophobizing for the hydrophobized inorganic powder in the oil phase. Specifically, the hydrophobic agent used may be silane or silicone, as a silicon-based hydrophobic agent, or a fluorine-based compound, an organic titanate compound, an amide or its salt, or a metal soap, hydrocarbon, higher fatty acid, higher alcohol, ester or wax, as a non-silicon-based hydrophobic agent. For use of a combination of hydrophobic agents, the specific examples and preferred examples for the components are the same as mentioned above.

The hydrophobized iron oxide powder and hydrophobized pigment-grade titanium oxide powder in the aqueous phase are preferably made by hydrophobizing non-hydrophobized powders with a hydrophobic agent containing one or more selected from the group consisting of dimethylpolysiloxane and methylhydrosiloxane-dimethylsiloxane copolymers, it being particularly preferred to use a hydrophobic agent containing a methylhydrosiloxane-dimethylsiloxane copolymer.

The degree of hydrophobic treatment of the hydrophobized inorganic powder to be added to the aqueous phase may be a minimum of 0.5 mass%, 0.75% mass% or 1 mass% up to a maximum of 5% mass%, 4 mass% or 3 mass%, based on the mass of the inorganic powder after it is hydrophobized. In other words, the degree of hydrophobic treatment of the hydrophobized inorganic powder may be from 0.5 to 5 mass%, from 0.5 to 4 mass%, from 0.5 to 3 mass%, from 0.75 to 5 mass%, from 0.75 to 4 mass%, from 0.75 to 3 mass%, from 1 to 5 mass%, from 1 to 4 mass% or preferably from 1 to 3 mass%.

The aqueous phase preferably contains a hydrophobized iron oxide powder and a hydrophobized pigment-grade titanium oxide powder where non-hydrophobized powders have been hydrophobized with a hydrophobic agent containing methylhydrogenpolysiloxane. It is most preferred to use a hydrophobized iron oxide powder and hydrophobized pigment-grade titanium oxide powder that have been hydrophobized with a hydrophobic agent containing methylhydrogenpolysiloxane with 1,3-butylene glycol and polyoxyethylen-methylpolysiloxane copolymer. Commercial products using hydrophobic agents include DIP T1 (hydrophobized pigment-grade titanium oxide powder), DIP R1 (red hydrophobized iron oxide powder), DIP Y1 (yellow hydrophobized iron oxide powder) and DIP K1 (black hydrophobized iron oxide powder), all by Sakai Chemical Industry Co., Ltd.

A mixture of several hydrophobized iron oxide powders may also be used as a component of the hydrophobized inorganic powder. For example, it may be a mixture of black hydrophobized iron oxide powder, red hydrophobized iron oxide powder and yellow hydrophobized iron oxide powder, with adjustment to a color tone to match the skin onto which it is to be applied.

The contents of black hydrophobized iron oxide powder, red hydrophobized iron oxide powder and yellow hydrophobized iron oxide powder may each be a minimum of 0.2 mass%, 0.5 mass% or 1 mass%, up to a maximum of 5 mass%, 3 mass% or 2 mass%, based on the total mass of the oil-in-water cosmetic. In other words, the powder contents may each be from 0.2 to 5 mass%, from 0.2 to 3 mass%, from 0.2 to 2 mass%, from 0.5 to 5 mass%, from 0.5 to 3 mass%, from 0.5 to 2 mass%, from 1 to 5 mass%, from 1 to 3 mass% or preferably from 1 to 2 mass%, based on the total mass of the oil-in-water cosmetic.

The content of the hydrophobized pigment-grade titanium oxide powder as a component of the hydrophobized inorganic powder may be a minimum of 3 mass%, 4 mass% or 5 mass%, up to a maximum of 19 mass%, 17 mass% or 15 mass%, based on the total mass of the oil-in-water cosmetic. In other words, the contents of the hydrophobized pigment-grade titanium oxide powders may each be from 3 to 19 mass%, from 3 to 17 mass%, from 3 to 15 mass%, from 4 to 19 mass%, from 4 to 17 mass%, from 4 to 15 mass%, from 5 to 19 mass%, from 5 to 17 mass% or preferably from 5 to 15 mass%, based on the total mass of the oil-in-water cosmetic. The content of the pigment-grade titanium oxide powder itself, subtracting the hydrophobized weight from the hydrophobized pigment-grade titanium oxide powder, may be greater than 1.85 mass% and less than 14.8 mass%, based on the total mass of the oil-in-water cosmetic.

The oil-in-water cosmetic can be used as a foundation, make-up base, concealer or sunscreen, or as a base composition for cosmetics. The form (shape and viscosity) may also be changed, or other components added, depending on the purpose. When it is to be used as a foundation, for example, the viscosity at 25°C at a rotational speed of 100 rpm, measured using a rotating viscometer, may be 5000 to 30,000 mPa· s, and preferably 10,000 to 20,000 mPa· s.

The oil-in-water cosmetic can be produced by a method that includes, for example, a step of obtaining a hydrophobized inorganic powder dispersed in an oil component, a step of stirring the oil component in an aqueous component to obtain an oil-in-water dispersion, and a step of adding a hydrophobized inorganic powder, which is the same type or a different type from the aforementioned hydrophobized inorganic powder, to the oil-in-water dispersion.

This method includes a first embodiment, wherein the fatty acid soap is added as a finished product to the oil component and/or aqueous component, and a second embodiment, wherein the fatty acid is added to the oil component, the basic compound is added to the aqueous component, and both are mixed to form the fatty acid soap.

The method of the first embodiment comprises the following steps (A1) to (A3):

(A1) a step of combining an oil containing a non-silicone oil, which has been heated, with a dispersion obtained by dispersing a hydrophobized inorganic powder in an oil, which is the same type or a different type from the aforementioned oil, by application of shearing force, and one or more nonionic surfactants having an overall HLB value of 6 to 15, to obtain an oil component in a heated state,

(A2) a step of stirring the oil component in a heated aqueous component containing an aminoalkylsulfonic acid polymer and an aqueous medium, to obtain an oil-in-water dispersion, and

(A3) a step of cooling the oil-in-water dispersion and adding a hydrophobized inorganic powder, which is the same type or a different type from the aforementioned hydrophobized inorganic powder, to add at least a portion thereof to the aqueous medium.

These steps are restricted by the following conditions (a1) to (a3):

(a1) the oil component and/or aqueous component contains a fatty acid soap,

(a2) the hydrophobized inorganic powders in the oil component and aqueous component contain hydrophobized iron oxide powder and hydrophobized pigment-grade titanium oxide powder, the content of the hydrophobized pigment-grade titanium oxide powder in the oil component being greater than 0.5 mass% and less than 5 mass% and the content of the hydrophobized pigment-grade titanium oxide powder in the aqueous component being greater than 2.5 mass% and less than 20 mass%, based on the total mass of the oil-in-water cosmetic, and

(a3) the total non-silicone oil content is greater than 5 mass% and less than 30 mass%, based on the total mass of the oil-in-water cosmetic.

In step (A1), the oil is heated to uniformity, at a heating temperature appropriately determined depending on the chemical species of the oil used, which will usually be in the range of 50 to 150°C, although the range of 50 to 100°C is preferred from the viewpoint of stability of the materials.

Step (A1) which yields the oil component in a heated state may be carried out with a heating temperature in the same temperature range as for heating of the oil.

When shearing force is to be applied to the oil in step (A1) to disperse the hydrophobized inorganic powder, the hydrophobized inorganic powder may be added to the oil and the shearing force applied using a triple roll mill disperser or bead mill disperser. The triple roll mill used may be an EXAKT 50I, EXACT 80E PLUS, EXACT 120EH-250 or EXACT 120EH-450, for example, by Nagase Screen Printing Research Co., Ltd.

The oil component is stirred in the heated aqueous component in step (A2), at a heating temperature that is in the same range as for heating of the silicone-based oil mentioned above. The stirring may be carried out using a homogenizer, for example, which may be an LR-1B or LR-2C by Mizuho Industrial Co., Ltd.

The oil-in-water dispersion is cooled in step (A3), which may be accomplished simply be allowing it to cool to room temperature (25°C, for example). The hydrophobized inorganic powder may be added to the oil-in-water dispersion by mixing them using a common stirrer.

The method of the second embodiment comprises the following steps (B1) to (B3):

(B1) a step of combining an oil containing a non-silicone oil, which has been heated, with a dispersion obtained by dispersing a hydrophobized inorganic powder in an oil, which is the same type or a different type from the aforementioned oil, by application of shearing force, one or more nonionic surfactants having an overall HLB value of 6 to 15, and a fatty acid, to obtain an oil component in a heated state,

(B2) a step of stirring the oil component in a heated aqueous component containing an aqueous medium, a basic compound to form a fatty acid soap by pairing with the carboxylic acid anion of the fatty acid, and an aminoalkylsulfonic acid polymer, to obtain an oil-in-water dispersion while forming the fatty acid soap, and

(B3) a step of cooling the oil-in-water dispersion and adding a hydrophobized inorganic powder, which is the same type or a different type from the aforementioned hydrophobized inorganic powder, to add at least a portion thereof to the aqueous medium.

These steps are restricted by the following conditions (b1) and (b2):

(b1) the hydrophobized inorganic powders in the oil component and aqueous component contain hydrophobized iron oxide powder and hydrophobized pigment-grade titanium oxide powder, the content of the hydrophobized pigment-grade titanium oxide powder in the oil component being greater than 0.5 mass% and less than 5 mass% and the content of the hydrophobized pigment-grade titanium oxide powder in the aqueous component being greater than 2.5 mass% and less than 20 mass%, based on the total mass of the oil-in-water cosmetic, and
(b2) the total non-silicone oil content is greater than 5 mass% and less than 30 mass%, based on the total mass of the oil-in-water cosmetic.

The heating in steps (B1) and (B2) may be carried out in the same manner as in steps (A1) and (A2) and the cooling in step (B3) may likewise be carried out as in step (A3), while the stirrer used may also be the same. The definitions, preferred examples and preferred ranges for the components in (a1) to (a3) and (b1) and (b2) are also as described for the embodiment of the oil-in-water cosmetic. The present invention also relates to a cosmetic process for caring for and/or making-up keratinic materials, comprising the application onto keratinic materials, in particular onto skin, of the oil-in-water cosmetic as defined in the invention. Advantageously, the oil-in-water cosmetic provides to the keratinic materials on which it is applied, (1) a fresh sensation, (2) a nourishing effect, (3) a coverage effect and (4) a lasting effect.

## Examples

The invention will now be illustrated by examples, with the understanding that the invention is not meant to be limited to these examples. Unless contrary indication, the % are expressed in mass% also referred as % by weight of the total mass (weight) of the oil-in-water cosmetic.

(Examples 1 to 8 and Comparative Examples 1 to 13)

Oil-in-water cosmetics were prepared by the following method.
Component A, comprising the ingredients listed as "A" in Tables 1 to 3, was heated to 75°C to obtain a homogeneous solution. Component B, comprising the ingredients listed as "B" in Tables 1 to 3, was uniformly dispersed with a triple roll mill disperser. The uniformly dispersed component B was then added to the homogeneously dissolved component A together with components C and D (comprising the components listed as "C" and "D", respectively, in Tables 1 to 3), and the temperature was adjusted to 75°C to prepare an oil component.

Separately, Components E, F and G, comprising the components listed as "E", "F" and "G", respectively, in Tables 1 to 3, were homogeneously dissolved and dispersed at room temperature, and heated to 75°C as the aqueous component. The oil component was added into the aqueous component and the mixture was emulsified using a homomixer. The emulsion was then cooled to room temperature, and components H and I, comprising the components listed as "H" and "I" in Tables 1 to 3, were added in that order and stirred to homogeneity, after which the mixture was filled into a container to prepare an oil-in-water cosmetic.
Table 4 shows the detailed information (product name, manufacturer, etc.) for each of the ingredients used. In Tables 5 to 7, the total oil contents (mass%), total non-silicone oil contents (mass%), hydrophobized pigment-grade titanium oxide powder contents (mass%) in oil phase, hydrophobized pigment-grade titanium oxide powder contents (mass%) in water phase, total titanium oxide powder (without the hydrophobic agents) contents (mass%), titanium oxide powder (without the hydrophobic agents) contents in the oil phase (mass%) and titanium oxide powder (without the hydrophobic agents) contents in the aqueous phase (mass%) are listed.

(Evaluation)

Stability evaluation:

Each prepared cosmetic was filled into a transparent container and sealed with a cap, and then stored for 1 month at 50°C. The condition of each cosmetic after storage was visually examined and compared with the product stored at room temperature (control). An assessment of "OK" was given when no separation, no powder aggregation or no crystal precipitation was observed, and an assessment of "NG" was given when any separation, powder aggregation or crystal precipitation was observed compared to the product stored at room temperature. The results are shown in Tables 5 to 7.

Organoleptic evaluation:

The fresh sensation, nourishing effect, coverage effect and lasting effect were evaluated in a single use test on skin by an evaluation panel of ten cosmetic experts (age 25-55), with evaluation on the following scale. The results are shown in Tables 5 to 7.

    A: Very excellent
    B: Excellent
    C: Poor
    D: Very poor

Viscosity:

The shear viscosity (mPa· s) of each cosmetic was measured at 25°C using a rotating viscometer (Rheolab QC by Anton Paar GmbH) (rotational speed: 100 rpm).

[Table 1]

| | | Component name | Example 1 | Example 2 | Example 3 | Example 4 | Example 5 | Example 6 | Example 7 | Example 8 |
|---|---|---|---|---|---|---|---|---|---|---|
| Oil phase | A | Triethylhexanoin*1 | 14.3 | 14.3 | 14.3 | 14.65 | 13.95 | 6.8 | 24.3 | - |
| | A | Vegetable squalane | - | - | - | - | - | - | - | 14.3 |
| | A | Dimethicone 20cs*2 | - | - | - | - | - | - | - | - |
| | A | Stearyl alcohol | 1 | 1 | 1 | 1 | 1 | 1 | 1 | 1 |
| | A | Self-emulsifiable glyceryl stearate*3 | 1 | 1 | 1 | 1 | 1 | 1 | 1 | 1 |
| | B | Black hydrophobized iron oxide (treated with isopropyltriisostearoyl titanate, sodium lauroyl aspartate and zinc chloride)*4 | 0.1 | 0.1 | 0.1 | 0.05 | 0.15 | 0.1 | 0.1 | 0.1 |
| | B | Red hydrophobized iron oxide (treated with isopropyltriisostearoyl titanate, sodium lauroyl aspartate and zinc chloride)*5 | 0.1 | 0.1 | 0.1 | 0.05 | 0.15 | 0.1 | 0.1 | 0.1 |
| | B | Yellow hydrophobized iron oxide (treated with isopropyltriisostearoyl titanate, sodium lauroyl aspartate and zinc chloride)*6 | 0.3 | 0.3 | 0.3 | 0.15 | 0.45 | 0.3 | 0.3 | 0.3 |
| | B | Hydrophobized pigment-grade titanium oxide (treated with isopropyltriisostearoyl titanate, sodium lauroyl aspartate and zinc chloride)*7 | 2.5 | 2.5 | 2.5 | 1.25 | 3.75 | 2.5 | 2.5 | 2.5 |
| | B | Triethylhexanoin*1 | 0.7 | 0.7 | 0.7 | 0.35 | 1.05 | 0.7 | 0.7 | - |
| | B | Vegetable squalane | - | - | - | - | - | - | - | 0.7 |
| | B | Dimethicone 20cs*2 | - | - | - | - | - | - | - | - |
| | B | Sorbitan sesquioleate (HLB=3.7)*8 | 0.1 | 0.1 | 0.1 | 0.05 | 0.15 | 0.1 | 0.1 | 0.1 |
| | C | Polysorbate 60 (HLB=14.9)*9 | 1 | 1 | 1 | 1 | 1 | 1 | 1 | 1 |
| | C | Sorbitan sesquioleate (HLB=3.7) | 0.9 | 0.9 | 0.9 | 0.95 | 0.85 | 0.9 | 0.9 | 0.9 |
| | D | Stearic acid*10 | 0.5 | 0.5 | 0.5 | 0.5 | 0.5 | 0.5 | 0.5 | 0.5 |

| | | Component name | Example 1 | Example 2 | Example 3 | Example 4 | Example 5 | Example 6 | Example 7 | Example 8 |
|---|---|---|---|---|---|---|---|---|---|---|
| Aqueous phase | E | Aminomethylpropanediol*11 | 0.3 | 0.3 | 0.3 | 0.3 | 0.3 | 0.3 | 0.3 | 0.3 |
| | F | Purified water | 46.4 | 52.8 | 40 | 47.9 | 44.9 | 53.9 | 36.4 | 46.4 |
| | F | Butylene glycol | 3 | 3 | 3 | 3 | 3 | 3 | 3 | 3 |
| | F | Phenoxyethanol | 0.5 | 0.5 | 0.5 | 0.5 | 0.5 | 0.5 | 0.5 | 0.5 |
| | F | Pentylene glycol | 2 | 2 | 2 | 2 | 2 | 2 | 2 | 2 |
| | G | (Acryloyldimethyltaurine ammonium/VP) copolymer*12 | 1.5 | 1.5 | 1.5 | 1.5 | 1.5 | 1.5 | 1.5 | 1.5 |
| | G | Carbomer*13 | - | - | - | - | - | - | - | - |
| | G | (Acrylate/alkyl acrylate (C10-30)) crosspolymer*14 | - | - | - | - | - | - | - | - |
| | H | Vinyldimethicone/methicone silsesquioxane crosspolymer, coconut fatty acid PEG-7 glyceryl, polyquatemium-7, methylsilanoltri(coconut fatty acid PEG-8 glyceryl)*15 | 3 | 3 | 3 | 3 | 3 | 3 | 3 | 3 |
| | H | Purified water | 3 | 3 | 3 | 3 | 3 | 3 | 3 | 3 |
| | I | Hydrophilized pigment-grade titanium oxide *16 | - | - | - | - | - | - | - | - |
| | I | Hydrophilized red iron oxide*17 | - | - | - | - | - | - | - | - |
| | I | Hydrophilized yellow iron oxide *18 | - | - | - | - | - | - | - | - |
| | I | Hydrophilized black iron oxide *19 | - | - | - | - | - | - | - | - |
| | I | Hydrophobized pigment-grade titanium oxide dispersion*20 | 10 | 5 | 15 | 10 | 10 | 10 | 10 | 10 |
| | I | Hydrophobized red iron oxide dispersion*21 | 0.8 | 0.4 | 1.2 | 0.8 | 0.8 | 0.8 | 0.8 | 0.8 |
| | I | Hydrophobized yellow iron oxide dispersion*22 | 1.6 | 0.8 | 2.4 | 1.6 | 1.6 | 1.6 | 1.6 | 1.6 |
| | I | Hydrophobized black iron oxide dispersion*23 | 0.4 | 0.2 | 0.6 | 0.4 | 0.4 | 0.4 | 0.4 | 0.4 |
| | I | Purified water | 5 | 5 | 5 | 5 | 5 | 5 | 5 | 5 |
| | | Total | 100 | 100 | 100 | 100 | 100 | 100 | 100 | 100 |

[Table 2]

| | | Component name | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|---|
| Oil phase | A | Triethylhexanoin*1 | 14.3 | 15 | 14.3 | 14.3 | 14.3 | 4.3 | 29.3 |
| | A | Vegetable squalane | - | - | - | - | - | - | - |
| | A | Dimethicone 20cs*2 | - | - | - | - | - | - | - |
| | A | Stearyl alcohol | 1 | 1 | 1 | 1 | 1 | 1 | 1 |
| | A | Self-emulsifiable glyceryl stearate*3 | 1 | 1 | 1 | 1 | 1 | 1 | 1 |
| | B | Black hydrophobized iron oxide (treated with isopropyltriisostearoyl titanate, sodium lauroyl aspartate and zinc chloride)*4 | 0.1 | - | 0.1 | 0.1 | 0.1 | 0.1 | 0.1 |
| | B | Red hydrophobized iron oxide (treated with isopropyltriisostearoyl titanate, sodium lauroyl aspartate and zinc chloride)*5 | 0.1 | - | 0.1 | 0.1 | 0.1 | 0.1 | 0.1 |
| | B | Yellow hydrophobized iron oxide (treated with isopropyltriisostearoyl titanate, sodium lauroyl aspartate and zinc chloride)*6 | 0.3 | - | 0.3 | 0.3 | 0.3 | 0.3 | 0.3 |
| | B | Hydrophobized pigment-grade titanium oxide (treated with isopropyltriisostearoyl titanate, sodium lauroyl aspartate and zinc chloride)*7 | 2.5 | - | 2.5 | 2.5 | 2.5 | 2.5 | 2.5 |
| | B | Triethylhexanoin*1 | 0.7 | - | 0.7 | 0.7 | 0.7 | 0.7 | 0.7 |
| | B | Vegetable squalane | - | - | - | - | - | - | - |
| | B | Dimethicone 20cs*2 | - | - | - | - | - | - | - |
| | B | Sorbitan sesquioleate (HLB=3.7)*8 | 0.1 | - | 0.1 | 0.1 | 0.1 | 0.1 | 0.1 |
| | C | Polysorbate 60 (HLB=14.9)*9 | 1 | 1 | 1 | 1 | 1 | 1 | 1 |
| | C | Sorbitan sesquioleate (HLB=3.7) | 0.9 | 1 | 0.9 | 0.9 | 0.9 | 0.9 | 0.9 |
| | D | Stearic acid*10 | 0.5 | 0.5 | - | 0.5 | 0.5 | 0.5 | 0.5 |
| Aqueous phase | E | Aminomethylpropanediol*11 | 0.3 | 0.3 | - | 0.3 | 0.3 | 0.3 | 0.3 |
| | F | Purified water | 48.76 | 49.4 | 47.2 | 46.4 | 46.4 | 56.4 | 31.4 |
| | F | Butylene glycol | 3 | 3 | 3 | 3 | 3 | 3 | 3 |
| | F | Phenoxyethanol | 0.5 | 0.5 | 0.5 | 0.5 | 0.5 | 0.5 | 0.5 |
| | F | Pentylene glycol | 2 | 2 | 2 | 2 | 2 | 2 | 2 |

(continued)

| | Component name | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|
| G | (Acryloyldimethyltaurine ammonium/VP)copolymer*12 | 1.5 | 1.5 | 1.5 | - | - | 1.5 | 1.5 |
| G | Carbomer *13 | - | - | - | 1.5 | - | - | - |
| G | (Acrylate/alkyl acrylate (C10-30)) crosspolymer *14 | - | - | - | - | 1.5 | - | - |
| H | Vinyldimethicone/methicone silsesquioxane crosspolymer, coconut fatty acid PEG-7 glyceryl, polyquaternium-7, methylsilanoltri(coconut fatty acid PEG-8 glyceryl)*15 | 3 | 3 | 3 | 3 | 3 | 3 | 3 |
| H | Purified water | 3 | 3 | 3 | 3 | 3 | 3 | 3 |
| I | Hydrophilized pigment-grade titanium oxide *16 | 8.4 | - | - | - | - | - | - |
| I | Hydrophilized red iron oxide*17 | 0.54 | - | - | - | - | - | - |
| I | Hydrophilized yellow iron oxide *18 | 1.04 | - | - | - | - | - | - |
| I | Hydrophilized black iron oxide *19 | 0.26 | - | - | - | - | - | - |
| I | Hydrophobized pigment-grade titanium oxide dispersion*20 | - | 10 | 10 | 10 | 10 | 10 | 10 |
| I | Hydrophobized red iron oxide dispersion*21 | - | 0.8 | 0.8 | 0.8 | 0.8 | 0.8 | 0.8 |
| I | Hydrophobized yellow iron oxide dispersion*22 | - | 1.6 | 1.6 | 1.6 | 1.6 | 1.6 | 1.6 |
| I | Hydrophobized black iron oxide dispersion*23 | - | 0.4 | 0.4 | 0.4 | 0.4 | 0.4 | 0.4 |
| I | Purified water | 5 | 5 | 5 | 5 | 5 | 5 | 5 |
| I | Total | 100 | 100 | 100 | 100 | 100 | 100 | 100 |

[Table 3]

| | | Component name | Comp. Ex. 8 | Comp. Ex. 9 | Comp. Ex. 10 | Comp. Ex. 11 | Comp. Ex. 12 | Comp. Ex. 13 |
|---|---|---|---|---|---|---|---|---|
| Oil phase | A | Triethylhexanoin*1 | - | 14.86 | 13.6 | 14.3 | 14.3 | 14.3 |
| | A | Vegetable squalane | - | - | - | - | - | - |
| | A | Dimethicone 20cs*2 | 14.3 | - | - | - | - | - |
| | A | Stearyl alcohol | 1 | 1 | 1 | 1 | 1 | 1 |
| | A | Self-emulsifiable glyceryl stearate*3 | 1 | 1 | 1 | 1 | 1 | 1 |
| | B | Black hydrophobized iron oxide (treated with isopropyltriisostearoyl titanate, sodium lauroyl aspartate and zinc chloride)*4 | 0.1 | 0.02 | 0.2 | 0.1 | 0.1 | 0.1 |
| | B | Red hydrophobized iron oxide (treated with isopropyltriisostearoyl titanate, sodium lauroyl aspartate and zinc chloride)*5 | 0.1 | 0.02 | 0.2 | 0.1 | 0.1 | 0.1 |
| | B | Yellow hydrophobized iron oxide (treated with isopropyltriisostearoyl titanate, sodium lauroyl aspartate and zinc chloride)*6 | 0.3 | 0.06 | 0.6 | 0.3 | 0.3 | 0.3 |
| | B | Hydrophobized pigment-grade titanium oxide (treated with isopropyltriisostearoyl titanate, sodium lauroyl aspartate and zinc chloride)*7 | 2.5 | 0.5 | 5 | 2.5 | 2.5 | 2.5 |
| | B | Triethylhexanoin*1 | - | 0.14 | 1.4 | 0.7 | 0.7 | 0.7 |
| | B | Vegetable squalane | - | - | - | - | - | - |
| | B | Dimethicone 20cs*2 | 0.7 | - | - | - | - | - |
| | B | Sorbitan sesquioleate (HLB=3.7)*8 | 0.1 | 0.02 | 0.2 | 0.1 | 0.1 | - |
| | C | Polysorbate 60 (HLB=14.9)*9 | 1 | 1 | 1 | 1 | 1 | - |
| | C | Sorbitan sesquioleate (HLB=3.7) | 0.9 | 0.98 | 0.8 | 0.9 | 0.9 | - |
| | D | Stearic acid*10 | 0.5 | 0.5 | 0.5 | 0.5 | 0.5 | 0.5 |

(continued)

| | | Component name | Comp. Ex. 8 | Comp. Ex. 9 | Comp. Ex. 10 | Comp. Ex. 11 | Comp. Ex. 12 | Comp. Ex. 13 |
|---|---|---|---|---|---|---|---|---|
| Aqueous phase | E | Aminomethylpropanediol*11 | 0.3 | 0.3 | 0.3 | 0.3 | 0.3 | 0.3 |
| | F | Purified water | 46.4 | 48.8 | 43.4 | 56 | 33.6 | 48.4 |
| | F | Butylene glycol | 3 | 3 | 3 | 3 | 3 | 3 |
| | F | Phenoxyethanol | 0.5 | 0.5 | 0.5 | 0.5 | 0.5 | 0.5 |
| | F | Pentylene glycol | 2 | 2 | 2 | 2 | 2 | 2 |
| | G | (Acryloyldimethyltaurine ammonium/VP) copolymer*12 | 1.5 | 1.5 | 1.5 | 1.5 | 1.5 | 1.5 |
| | G | Carbomer *13 | - | - | - | - | - | - |
| | G | (Acrylate/alkyl acrylate (C10-30)) crosspolymer *14 | - | - | - | - | - | - |
| | H | Vinyldimethicone/methicone silsesquioxane crosspolymer, coconut fatty acid PEG-7 glyceryl, polyquaternium-7, methylsilanoltri (coconut fatty acid PEG-8 glyceryl)*15 | 3 | 3 | 3 | 3 | 3 | 3 |
| | H | Purified water | 3 | 3 | 3 | 3 | 3 | 3 |
| | I | Hydrophilized pigment-grade titanium oxide *16 | - | - | - | - | - | - |
| | I | Hydrophilized red iron oxide*17 | - | - | - | - | - | - |
| | I | Hydrophilized yellow iron oxide *18 | - | - | - | - | - | - |
| | I | Hydrophilized black iron oxide *19 | - | - | - | - | - | - |
| | I | Hydrophobized pigment-grade titanium oxide dispersion*20 | 10 | 10 | 10 | 2.5 | 20 | 10 |
| | I | Hydrophobized red iron oxide dispersion*21 | 0.8 | 0.8 | 0.8 | 0.2 | 1.6 | 0.8 |
| | I | Hydrophobized yellow iron oxide dispersion*22 | 1.6 | 1.6 | 1.6 | 0.4 | 3.2 | 1.6 |
| | I | Hydrophobized black iron oxide dispersion*23 | 0.4 | 0.4 | 0.4 | 0.1 | 0.8 | 0.4 |
| | I | Purified water | 5 | 5 | 5 | 5 | 5 | 5 |
| | | Total | 100 | 100 | 100 | 100 | 100 | 100 |

[Table 4]

| | Trade name (Manufacturer) |
|---|---|
| *1 | CRODAMOL GTEH-LQ (Croda) |
| *2 | XIAMETER PMX-200 SILICONE FLUID 20CS (Toray Dow Coming) |
| *3 | TEGIN PELLETS (Evonik) |
| *4 | C 337001 SUN PURO BLACK IRON OXIDE ASI (Daito Kasei Kogyo) |
| *5 | C 338001 SUN PURO RED IRON OXIDE ASI (Daito Kasei Kogyo) |

(continued)

|  | Trade name (Manufacturer) |
|---|---|
| *6 | C 339001 SUN PURO YELLOW IRON OXIDE ASI (Daito Kasei Kogyo) |
| *7 | TIO2 CR-50 ASI (Daito Kasei Kogyo) |
| *8 | SPAN 83 (Croda) |
| *9 | TWEEN 60 (Croda) |
| *10 | PARMERA 9818 (Klkoleo) |
| *11 | AMPD ULTRA PC (Angus Chemical) |
| *12 | ARISTOFREX AVC (Clariant) |
| *13 | CARBOPOL 980 (Lubrizol) |
| *14 | PEMUREN TR-1 (Lubrizol) |
| *15 | MW SRP 100 (Miyoshi Kasei) |
| *16 | SYMPHOLIGHT WW (JGC Catalysts and Chemicals) |
| *17 | SYMPHOLIGHT RW (JGC Catalysts and Chemicals) |
| *18 | SYMPHOLIGHT YW (JGC Catalysts and Chemicals) |
| *19 | SYMPHOLIGHT BW (JGC Catalysts and Chemicals) |
| *20 | DIP-T1 (Sakai Chemical Industry) |
| *21 | DIP-R1 (Sakai Chemical Industry) |
| *22 | DIP-Y1 (Sakai Chemical Industry) |
| *23 | DIP-K1 (Sakai Chemical Industry) |

[Table 5]

| | Example 1 | Example 2 | Example 3 | Example 4 | Example 5 | Example 6 | Example 7 | Example 8 |
|---|---|---|---|---|---|---|---|---|
| Total amount of oil | 15 | 15 | 15 | 15 | 15 | 7.5 | 25 | 15 |
| Total amount of non-silicone oil | 15 | 15 | 15 | 15 | 15 | 7.5 | 25 | 15 |
| Hydrophobized pigment-grade $TiO_2$ amount in oil phase | 2.5 | 2.5 | 2.5 | 1.25 | 3.75 | 2.5 | 2.5 | 2.5 |
| Hydrophobized pigment-grade $TiO_2$ amount in water phase | 10 | 5 | 15 | 10 | 10 | 10 | 10 | 10 |
| Total $TiO_2$ (without hydrophobic agent) amount in oil and aqueous phases | 9.75 | 6.05 | 13.45 | 8.575 | 10.925 | 9.75 | 9.75 | 9.75 |
| $TiO_2$ (without hydrophobic agent) amount in oil phase | 2.35 | 2.35 | 2.35 | 1.175 | 3.525 | 2.35 | 2.35 | 2.35 |
| $TiO_2$ (without hydrophobic agent) amount in aqueous phase | 7.4 | 3.7 | 11.1 | 7.4 | 7.4 | 7.4 | 7.4 | 7.4 |
| Viscosity (mPa-s) | 14680 | 11780 | 18205 | 13000 | 15140 | 11540 | 14800 | 11230 |
| Stability (50°C 1M) | OK | OK | OK | OK | OK | OK | OK | OK |
| Fresh sensation | A | A | B | A | B | A | B | B |
| Nourishing effect | A | A | B | A | B | B | A | B |
| Coverage effect | A | B | A | B | A | A | A | B |
| Lasting effect | A | A | A | A | A | A | B | B |

[Table 6]

| | Comparative Example 1 | Comparative Example 2 | Comparative Example 3 | Comparative Example 4 | Comparative Example 5 | Comparative Example 6 | Comparative Example 7 |
|---|---|---|---|---|---|---|---|
| Total amount of oil | 15 | 15 | 15 | 15 | 15 | 5 | 30 |
| Total amount of non-silicone oil | 15 | 15 | 15 | 15 | 15 | 5 | 30 |
| Hydrophobized pigment-grade $TiO_2$ amount in oil phase | 2.5 | 0 | 2.5 | 2.5 | 2.5 | 2.5 | 2.5 |
| Hydrophobized pigment-grade $TiO_2$ amount in water phase | 0 | 10 | 10 | 10 | 10 | 10 | 10 |
| Total $TiO_2$ (without hydrophobic agent) amount in oil and aqueous phases | 9.75 | 7.4 | 9.75 | 9.75 | 9.75 | 9.75 | 9.75 |
| $TiO_2$ (without hydrophobic agent) amount in oil phase | 2.35 | 0 | 2.35 | 2.35 | 2.35 | 2.35 | 2.35 |
| $TiO_2$ (without hydrophobic agent) amount in aqueous phase | 7.4 | 7.4 | 7.4 | 7.4 | 7.4 | 7.4 | 7.4 |
| Viscosity (mPa-s) | 18000 | 13680 | 13080 | 11670 | 9087 | 11980 | 6623 |
| Stability (50°C 1M) | OK | OK | NG | NG | NG | OK | NG |
| Fresh sensation | A | A | A | D | D | A | D |
| Nourishing effect | A | A | A | D | D | D | A |
| Coverage effect | C | D | C | D | D | A | A |
| Lasting effect | D | D | C | D | D | A | B |

EP 3 897 517 B1

[Table 7]

| | Comparative Example 8 | Comparative Example 9 | Comparative Example 10 | Comparative Example 11 | Comparative Example 12 | Comparative Example 13 |
|---|---|---|---|---|---|---|
| Total amount of oil | 15 | 15 | 15 | 15 | 15 | 15 |
| Total amount of non-silicone oil | 0 | 15 | 15 | 15 | 15 | 15 |
| Hydrophobized pigment-grade $TiO_2$ amount in oil phase | 2.5 | 0.5 | 5 | 2.5 | 2.5 | 2.5 |
| Hydrophobized pigment-grade $TiO_2$ amount in water phase | 10 | 10 | 10 | 2.5 | 20 | 10 |
| Total $TiO_2$ (without hydrophobic agent) amount in oil and aqueous phases | 9.75 | 7.87 | 12.1 | 4.2 | 17.15 | 9.75 |
| $TiO_2$ (without hydrophobic agent) amount in oil phase | 2.35 | 0.47 | 4.7 | 2.35 | 2.35 | 2.35 |
| $TiO_2$ (without hydrophobic agent) amount in aqueous phase | 7.4 | 7.4 | 7.4 | 1.85 | 14.8 | 7.4 |
| Viscosity (mPa-s) | 10430 | 9010 | 15970 | 11680 | 14470 | 17930 |
| Stability (50°C 1M) | NG | OK | NG | OK | OK | NG |
| Fresh sensation | D | A | D | A | D | C |
| Nourishing effect | D | A | D | A | D | C |
| Coverage effect | B | C | A | C | A | A |
| Lasting effect | D | C | A | C | A | A |

All these results show that the oil-in-water cosmetic of the invention, comprising a combination of specific compounds and specific mass% ranges, provides improved properties in terms of (1) a fresh sensation, (2) a nourishing effect, (3) a coverage effect and (4) a lasting effect, in comparison to all comparatives examples which do not have the key features of the oil-in-water cosmetic of the invention (ie either they do not contain a key compound of the oil-in-water cosmetic of the invention, either the mass% ranges are outside of the claimed mass% ranges).

**Claims**

1. An oil-in-water cosmetic composed of an aqueous phase and an oil phase, and comprising hydrophobized inorganic powder, a fatty acid soap, a nonionic surfactant and a hydrophilic polymer, wherein

   the aqueous phase includes an aqueous medium while the oil phase includes an oil containing a non-silicone oil, the hydrophobized inorganic powder includes at least hydrophobized iron oxide powder and hydrophobized pigment-grade titanium oxide powder, with hydrophobized inorganic powder of the same type or different types being present in the oil phase and the aqueous phase,
   the contents of the hydrophobized pigment-grade titanium oxide powder in the oil phase and in the aqueous phase are, respectively, greater than 0.5 mass% and less than 5 mass%, and greater than 2.5 mass% and less than 20 mass%, based on the total mass of the oil-in-water cosmetic,
   one or more nonionic surfactants are included, with an overall HLB value of 6 to 15,
   the hydrophilic polymer contains a hydrophilic polymer including, as a repeating unit, an ethylenic unsaturated compound having an aminoalkylsulfonic acid or salt thereof as a substituent, and
   the total non-silicone oil content is greater than 5 mass% and less than 30 mass%, based on the total mass of the oil-in-water cosmetic.

2. The oil-in-water cosmetic according to claim 1, wherein
   the hydrophobized iron oxide powder and hydrophobized pigment-grade titanium oxide powder are hydrophobized by a hydrophobic agent containing one or more selected from the group consisting of trialkoxyalkylsilane, dimethylpolysiloxane, methylhydrosiloxane-dimethylsiloxane copolymer, a salt of amide derived from higher fatty acid and $\alpha$-amino acid, and alkyl titanate.

3. The oil-in-water cosmetic according to claim 1 or 2, wherein the hydrophobized iron oxide powder and hydrophobized pigment-grade titanium oxide powder in the oil phase are hydrophobized by a hydrophobic agent containing one or more selected from the group consisting of a salt of amide derived from higher fatty acid and $\alpha$-amino acid, alkyl titanate, and methylhydrosiloxane-dimethylsiloxane copolymer.

4. The oil-in-water cosmetic according to claim 1 or 2, wherein the hydrophobized iron oxide powder and hydrophobized pigment-grade titanium oxide powder in the aqueous phase are hydrophobized by a hydrophobic agent containing one or more selected from the group consisting of dimethylpolysiloxane and methylhydrosiloxane-dimethylsiloxane copolymer.

5. The oil-in-water cosmetic according to any one of claims 1 to 4, wherein the non-silicone oil is an ester oil and/or a hydrocarbon oil.

6. The oil-in-water cosmetic according to any one of claims 1 to 5, wherein the oil-in-water cosmetic is used as a foundation, a make-up base, a concealer or a sunscreen.

7. A method for producing an oil-in-water cosmetic comprising:

   a step of combining an oil containing a non-silicone oil, which has been heated, with a dispersion obtained by dispersing a hydrophobized inorganic powder in an oil, which is the same type or a different type from the aforementioned oil, by application of shearing force, and one or more nonionic surfactants having an overall HLB value of 6 to 15, to obtain an oil component in a heated state,
   a step of stirring the oil component in a heated aqueous component containing an aqueous medium and a hydrophilic polymer including, as a repeating unit, an ethylenic unsaturated compound having an aminoalkylsulfonic acid or salt thereof as a substituent, to obtain an oil-in-water dispersion, and
   a step of cooling the oil-in-water dispersion and adding a hydrophobized inorganic powder, which is the same type or a different type from the aforementioned hydrophobized inorganic powder, to add at least a portion thereof to the aqueous medium, wherein
the oil component and/or aqueous component contains a fatty acid soap,
the hydrophobized inorganic powders in the oil component and aqueous component contain hydrophobized iron oxide powder and hydrophobized pigment-grade titanium oxide powder, the content of the hydrophobized pigment-grade titanium oxide powder in the oil component being greater than 0.5 mass% and less than 5 mass% and the content of the hydrophobized pigment-grade titanium oxide powder in the aqueous component being greater than 2.5 mass% and less than 20 mass%, based on the total mass of the oil-in-water cosmetic, and the total non-silicone oil content is greater than 5 mass% and less than 30 mass%, based on the total mass of the oil-in-water cosmetic.

8. A method for producing an oil-in-water cosmetic comprising:

a step of combining an oil containing a non-silicone oil, which has been heated, with a dispersion obtained by dispersing a hydrophobized inorganic powder in an oil, which is the same type or a different type from the aforementioned oil, by application of shearing force, one or more nonionic surfactants having an overall HLB value of 6 to 15, and a fatty acid, to obtain an oil component in a heated state,
a step of stirring the oil component in a heated aqueous component containing an aqueous medium, a basic compound to form a fatty acid soap by pairing with the carboxylic acid anion of the fatty acid, and a hydrophilic polymer including, as a repeating unit, an ethylenic unsaturated compound having an aminoalkylsulfonic acid or salt thereof as a substituent, to obtain an oil-in-water dispersion while forming the fatty acid soap, and
a step of cooling the oil-in-water dispersion and adding a hydrophobized inorganic powder, which is the same type or a different type from the aforementioned hydrophobized inorganic powder, to add at least a portion thereof to the aqueous medium, wherein
the hydrophobized inorganic powders in the oil component and aqueous component contain hydrophobized iron oxide powder and hydrophobized pigment-grade titanium oxide powder, the content of the hydrophobized pigment-grade titanium oxide powder in the oil component being greater than 0.5 mass% and less than 5 mass% and the content of the hydrophobized pigment-grade titanium oxide powder in the aqueous component being greater than 2.5 mass% and less than 20 mass%, based on the total mass of the oil-in-water cosmetic, and the total non-silicone oil content is greater than 5 mass% and less than 30 mass%, based on the total mass of the oil-in-water cosmetic.

9. The method according to claim 7 or 8, wherein the oil to which the dispersion is added comprises an emulsification aid.

10. The method according to any one of claims 7 to 9, wherein the dispersion comprises a dispersing agent.

11. An oil-in-water cosmetic that is obtainable by the method according to any one of claims 7 to 10.

12. A cosmetic process for caring for and/or making-up keratinic materials, comprising the application onto keratinic materials, in particular onto skin, of the oil-in-water cosmetic as defined in anyone of claims 1 to 6 and 11.

13. Use of the oil-in-water cosmetic as defined in anyone of claims 1 to 6 and 11 for providing to the keratinic materials on which it is applied, a fresh sensation, a nourishing effect, a coverage effect and a long lasting effect.

**Patentansprüche**

1. Öl-in-Wasser-Kosmetik, die aus einer wässrigen Phase und einer Ölphase zusammengesetzt ist und hydrophobiertes anorganisches Pulver, eine Fettsäureseife, ein nichtionisches Tensid und ein hydrophiles Polymer umfasst, wobei

die wässrige Phase ein wässriges Medium beinhaltet, während die Ölphase ein Öl beinhaltet, das ein Nicht-Silikonöl enthält,
das hydrophobierte anorganische Pulver mindestens hydrophobiertes Eisenoxidpulver und hydrophobiertes Titanoxidpulver in Pigmentqualität beinhaltet, wobei in der Ölphase und der wässrigen Phase hydrophobiertes anorganisches Pulver des gleichen Typs oder unterschiedlicher Typen vorhanden ist,
die Gehalte des hydrophobierten Titanoxidpulvers in Pigmentqualität in der Ölphase und in der wässrigen Phase, auf die Gesamtmasse der Öl-in-Wasser-Kosmetik bezogen, größer als 0,5 Massen-% und kleiner als 5 Massen-% bzw. größer als 2,5 Massen-% und kleiner als 20 Massen-% sind,
ein oder mehrere nichtionische Tenside beinhaltet sind, mit einem Gesamt-HLB-Wert von 6 bis 15,

das hydrophile Polymer ein hydrophiles Polymer enthält, das als eine sich wiederholende Einheit eine ethylenisch ungesättigte Verbindung mit einer Aminoalkylsulfonsäure oder einem Salz davon als Substituent beinhaltet, und der Gesamtgehalt an Nicht-Silikonöl, auf die Gesamtmasse der Öl-in-Wasser-Kosmetik bezogen, größer als 5 Massen-% und kleiner als 30 Massen-% ist.

2.  Öl-in-Wasser-Kosmetik nach Anspruch 1, wobei
das hydrophobierte Eisenoxidpulver und das hydrophobierte Titanoxidpulver in Pigmentqualität durch ein hydrophobes Mittel hydrophobiert sind, das eines oder mehrere enthält, ausgewählt aus der Gruppe bestehend aus Trialkoxyalkylsilan, Dimethylpolysiloxan, Methylhydrosiloxan-Dimethylsiloxan-Copolymer, einem Salz von Amid, das von höherer Fettsäure und $\alpha$-Aminosäure abgeleitet ist, und Alkyltitanat.

3.  Öl-in-Wasser-Kosmetik nach Anspruch 1 oder 2, wobei das hydrophobierte Eisenoxidpulver und das hydrophobierte Titanoxidpulver in Pigmentqualität in der Ölphase durch ein hydrophobes Mittel hydrophobiert sind, das eines oder mehrere enthält, ausgewählt aus der Gruppe bestehend aus einem Salz von Amid, das von höherer Fettsäure und $\alpha$-Aminosäure abgeleitet ist, Alkyltitanat, und Methylhydrosiloxan-Dimethylsiloxan-Copolymer.

4.  Öl-in-Wasser-Kosmetik nach Anspruch 1 oder 2, wobei das hydrophobierte Eisenoxidpulver und das hydrophobierte Titanoxidpulver in Pigmentqualität in der wässrigen Phase durch ein hydrophobes Mittel hydrophobiert sind, das eines oder mehrere enthält, ausgewählt aus der Gruppe bestehend aus Dimethylpolysiloxan und Methylhydrosiloxan-Dimethylsiloxan-Copolymer.

5.  Öl-in-Wasser-Kosmetik nach einem der Ansprüche 1 bis 4, wobei es sich bei dem Nicht-Silikonöl um ein Esteröl und/oder ein Kohlenwasserstofföl handelt.

6.  Öl-in-Wasser-Kosmetik nach einem der Ansprüche 1 bis 5, wobei die Öl-in-Wasser-Kosmetik als Grundierung, Make-up-Basis, Abdeckprodukt oder Sonnenschutz verwendet wird.

7.  Verfahren zur Herstellung einer Öl-in-Wasser-Kosmetik, umfassend:

einen Schritt des Kombinierens eines Öls, das ein Nicht-Silikonöl enthält, welches erhitzt wurde, mit einer Dispersion, die durch Dispergieren eines hydrophobierten anorganischen Pulvers in einem Öl, das vom gleichen Typ oder einem anderen Typ ist als das vorstehend genannte Öl, durch Anwendung von Scherkraft erhalten wurde, und einem oder mehreren nichtionischen Tensiden mit einem Gesamt-HLB-Wert von 6 bis 15, um eine Ölkomponente in einem erhitzten Zustand zu erhalten,
einen Schritt des Rührens der Ölkomponente in einer erhitzten wässrigen Komponente, die ein wässriges Medium und ein hydrophiles Polymer enthält, das als eine sich wiederholende Einheit eine ethylenisch ungesättigte Verbindung mit einer Aminoalkylsulfonsäure oder einem Salz davon als Substituent beinhaltet, um eine Öl-in-Wasser-Dispersion zu erhalten, und
einen Schritt des Abkühlens der Öl-in-Wasser-Dispersion und des Zugebens eines hydrophobierten anorganischen Pulvers, das vom gleichen Typ oder einem anderen Typ ist als das vorstehend genannte hydrophobierte anorganische Pulver, um mindestens einen Teil davon dem wässrigen Medium zuzugeben, wobei
die Ölkomponente und/oder die wässrige Komponente eine Fettsäureseife enthält,
die hydrophobierten anorganischen Pulver in der Ölkomponente und der wässrigen Komponente hydrophobiertes Eisenoxidpulver und hydrophobiertes Titanoxidpulver in Pigmentqualität enthalten, wobei, auf die Gesamtmasse der Öl-in-Wasser-Kosmetik bezogen, der Gehalt des hydrophobierten Titanoxidpulvers in Pigmentqualität in der Ölkomponente größer als 0,5 Massen-% und kleiner als 5 Massen-% ist und der Gehalt des hydrophobierten Titanoxidpulvers in Pigmentqualität in der wässrigen Komponente größer als 2,5 Massen-% und kleiner als 20 Massen-% ist, und
der Gesamtgehalt an Nicht-Silikonöl, auf die Gesamtmasse der Öl-in-Wasser-Kosmetik bezogen, größer als 5 Massen-% und kleiner als 30 Massen-% ist.

8.  Verfahren zur Herstellung eine Öl-in-Wasser-Kosmetik, umfassend:

einen Schritt des Kombinierens eines Öls, das ein Nicht-Silikonöl enthält, welches erhitzt wurde, mit einer Dispersion, die durch Dispergieren eines hydrophobierten anorganischen Pulvers in einem Öl, das vom gleichen Typ oder einem anderen Typ ist als das vorstehend genannte Öl, durch Anwendung von Scherkraft erhalten wurde, einem oder mehreren nichtionischen Tensiden mit einem Gesamt-HLB-Wert von 6 bis 15, und einer Fettsäure, um eine Ölkomponente in einem erhitzten Zustand zu erhalten,

einen Schritt des Rührens der Ölkomponente in einer erhitzten wässrigen Komponente, die ein wässriges Medium, eine basische Verbindung, um durch Paarung mit dem Carbonsäureanion der Fettsäure eine Fettsäureseife zu bilden, und ein hydrophiles Polymer enthält, das als eine sich wiederholende Einheit eine ethylenisch ungesättigte Verbindung mit einer Aminoalkylsulfonsäure oder einem Salz davon als Substituent beinhaltet, um eine Öl-in-Wasser-Dispersion zu erhalten, während die Fettsäureseife gebildet wird, und
einen Schritt des Abkühlens der Öl-in-Wasser-Dispersion und des Zugebens eines hydrophobierten anorganischen Pulvers, das vom gleichen Typ oder einem anderen Typ ist als das vorstehend genannte hydrophobierte anorganische Pulver, um mindestens einen Teil davon dem wässrigen Medium zuzugeben, wobei
die hydrophobierten anorganischen Pulver in der Ölkomponente und der wässrigen Komponente hydrophobiertes Eisenoxidpulver und hydrophobiertes Titanoxidpulver in Pigmentqualität enthalten, wobei, auf die Gesamtmasse der Öl-in-Wasser-Kosmetik bezogen, der Gehalt des hydrophobierten Titanoxidpulvers in Pigmentqualität in der Ölkomponente größer als 0,5 Massen-% und kleiner als 5 Massen-% ist und der Gehalt des hydrophobierten Titanoxidpulvers in Pigmentqualität in der wässrigen Komponente größer als 2,5 Massen-% und kleiner als 20 Massen-% ist, und
der Gesamtgehalt an Nicht-Silikonöl, auf die Gesamtmasse der Öl-in-Wasser-Kosmetik bezogen, größer als 5 Massen-% und kleiner als 30 Massen-% ist.

9. Verfahren nach Anspruch 7 oder 8, wobei das Öl, dem die Dispersion zugegeben wird, eine Emulgierhilfe umfasst.

10. Verfahren nach einem der Ansprüche 7 bis 9, wobei die Dispersion ein Dispergiermittel umfasst.

11. Öl-in-Wasser-Kosmetik, die durch das Verfahren nach einem der Ansprüche 7 bis 10 erhalten werden kann.

12. Kosmetischer Prozess zum Pflegen und/oder Schminken von keratinischen Materialien, der das Auftragen der Öl-in-Wasser-Kosmetik, wie in einem der Ansprüche 1 bis 6 und 11 definiert, auf keratinische Materialien, insbesondere auf Haut, umfasst.

13. Verwendung des Öl-in-Wasser-Kosmetik, wie in einem der Ansprüche 1 bis 6 und 11 definiert, um den keratinischen Materialien, auf die es aufgetragen wird, ein Frischegefühl, einen nährenden Effekt, einen deckenden Effekt und einen langanhaltenden Effekt zu bieten.

**Revendications**

1. Produit cosmétique huile dans eau composé d'une phase aqueuse et d'une phase huileuse, et comprenant une poudre inorganique rendue hydrophobe, un savon d'acide gras, un tensioactif non ionique et un polymère hydrophile, dans lequel

la phase aqueuse comporte un milieu aqueux tandis que la phase huileuse comporte une huile contenant une huile sans silicone,
la poudre inorganique rendue hydrophobe comporte au moins de la poudre d'oxyde de fer rendue hydrophobe et de la poudre d'oxyde de titane de qualité pigment rendue hydrophobe, de la poudre inorganique rendue hydrophobe du même type ou de différents types étant présente dans la phase huileuse et la phase aqueuse, les teneurs en la poudre d'oxyde de titane de qualité pigment rendue hydrophobe dans la phase huileuse et dans la phase aqueuse sont, respectivement, supérieure à 0,5 % en masse et inférieure à 5 % en masse, et supérieure à 2,5 % en masse et inférieure à 20 % en masse, sur la base de la masse totale du produit cosmétique huile dans eau,
un ou plusieurs tensioactifs non ioniques sont inclus, avec une valeur HLB globale de 6 à 15,
le polymère hydrophile contient un polymère hydrophile comportant, comme motif de répétition, un composé insaturé éthylénique présentant un acide aminoalkylsulfonique ou un sel de celui-ci en tant que substituant, et
la teneur totale en huile sans silicone est supérieure à 5 % en masse et inférieure à 30 % en masse, sur la base de la masse totale du produit cosmétique huile dans eau.

2. Produit cosmétique huile dans eau selon la revendication 1, dans lequel
la poudre d'oxyde de fer rendue hydrophobe et la poudre d'oxyde de titane de qualité pigment rendue hydrophobe sont rendues hydrophobes par un agent hydrophobe contenant un ou plusieurs choisis dans le groupe constitué de trialcoxyalkysilane, de diméthylpolysiloxane, d'un copolymère méthylhydrosiloxane-diméthylsiloxane, d'un sel d'amide dérivé d'un acide gras supérieur et d'un acide $\alpha$-aminé, et de titanate d'alkyle.

3. Produit cosmétique huile dans eau selon la revendication 1 ou 2, dans lequel la poudre d'oxyde de fer rendue hydrophobe et la poudre d'oxyde de titane de qualité pigment rendue hydrophobe dans la phase huileuse sont rendues hydrophobes par un agent hydrophobe contenant un ou plusieurs choisis dans le groupe constitué d'un sel d'amide dérivé d'un acide gras supérieur et d'un acide $\alpha$-aminé, et de titanate d'alkyle, et d'un copolymère méthylhydrosiloxane-diméthylsiloxane.

4. Produit cosmétique huile dans eau selon la revendication 1 ou 2, dans lequel la poudre d'oxyde de fer rendue hydrophobe et la poudre d'oxyde de titane de qualité pigment rendue hydrophobe dans la phase aqueuse sont rendues hydrophobes par un agent hydrophobe contenant un ou plusieurs choisis dans le groupe constitué de diméthylpolysiloxane et d'un copolymère méthylhydrosiloxane-diméthylsiloxane.

5. Produit cosmétique huile dans eau selon l'une quelconque des revendications 1 à 4, dans lequel l'huile sans silicone est une huile d'ester et/ou une huile hydrocarbonée.

6. Produit cosmétique huile dans eau selon l'une quelconque des revendications 1 à 5, dans lequel le produit cosmétique huile dans eau est utilisé comme fond de teint, base de maquillage, correcteur ou écran solaire.

7. Procédé pour produire un produit cosmétique huile dans eau comprenant :

une étape de combinaison d'une huile contenant une huile sans silicone, qui a été chauffée, avec une dispersion obtenue par dispersion d'une poudre inorganique rendue hydrophobe dans une huile, qui est du même type ou d'un type différent de l'huile susmentionnée, par application d'une force de cisaillement, et un ou plusieurs tensioactifs non ioniques présentant une valeur HLB globale de 6 à 15, pour obtenir un composant d'huile dans un état chauffé,

une étape d'agitation du composant huile dans un composant aqueux chauffé contenant un milieu aqueux et un polymère hydrophile comportant, comme motif de répétition, un composé insaturé éthylénique présentant un acide aminoalkylsulfonique ou sel de celui-ci en tant que substituant, pour obtenir une dispersion huile dans eau, et

une étape de refroidissement de la dispersion huile dans eau et d'ajout d'une poudre inorganique rendue hydrophobe, qui est du même type ou d'un type différent de la poudre inorganique rendue hydrophobe susmentionnée, pour ajouter au moins une partie de celle-ci au milieu aqueux, dans lequel,

le composant huile et/ou composant aqueux contient un savon d'acide gras,

les poudres inorganiques rendues hydrophobes dans le composant huile et composant aqueux contiennent de la poudre d'oxyde de fer rendue hydrophobe et de la poudre d'oxyde de titane de qualité pigment rendue hydrophobe, la teneur en poudre d'oxyde de titane de qualité pigment rendue hydrophobe dans le composant huile étant supérieure à 0,5 % en masse et inférieure à 5 % en masse et la teneur en poudre d'oxyde de titane de qualité pigment rendue hydrophobe dans le composant aqueux étant supérieure à 2,5 % en masse et inférieure à 20 % en masse, sur la base de la masse totale du produit cosmétique huile dans eau, et

la teneur totale en huile sans silicone est supérieure à 5 % en masse et inférieure à 30 % en masse, sur la base de la masse totale du produit cosmétique huile dans eau.

8. Procédé pour produire un produit cosmétique huile dans eau comprenant :

une étape de combinaison d'une huile contenant une huile sans silicone, qui a été chauffée, avec une dispersion obtenue par dispersion d'une poudre inorganique rendue hydrophobe dans une huile, qui est du même type ou d'un type différent de l'huile susmentionnée, par application d'une force de cisaillement, un ou plusieurs tensioactifs non ioniques présentant une valeur HLB globale de 6 à 15, et un acide gras, pour obtenir un composant huile dans un état chauffé,

une étape d'agitation du composant huile dans un composant aqueux chauffé contenant un milieu aqueux, un composé basique pour former un savon d'acide gras par appariement avec l'anion d'acide carboxylique de l'acide gras, et un polymère hydrophile comportant, comme motif de répétition, un composé insaturé éthylénique présentant un acide aminoalkylsulfonique ou sel de celui-ci en tant que substituant, pour obtenir une dispersion huile dans eau tout en formant le savon d'acide gras, et

une étape de refroidissement de la dispersion huile dans eau et d'ajout d'une poudre inorganique rendue hydrophobe, qui est du même type ou d'un type différent de la poudre inorganique rendue hydrophobe susmentionnée, pour ajouter au moins une partie de celle-ci au milieu aqueux, dans lequel,

les poudres inorganiques rendues hydrophobes dans le composant huile et composant aqueux contiennent de la poudre d'oxyde de fer rendue hydrophobe et de la poudre d'oxyde de titane de qualité pigment rendue hydrophobe, la teneur en poudre d'oxyde de titane de qualité pigment rendue hydrophobe dans le composant huile étant supérieure à 0,5 % en masse et inférieure à 5 % en masse et la teneur en poudre d'oxyde de titane de qualité pigment rendue hydrophobe dans le composant aqueux étant supérieure à 2,5 % en masse et inférieure à 20 % en masse, sur la base de la masse totale du produit cosmétique huile dans eau, et la teneur totale en huile sans silicone est supérieure à 5 % en masse et inférieure à 30 % en masse, sur la base de la masse totale du produit cosmétique huile dans eau.

9. Procédé selon la revendication 7 ou 8, dans lequel l'huile à laquelle la dispersion est ajoutée comprend un auxiliaire d'émulsification.

10. Procédé selon l'une quelconque des revendications 7 à 9, dans lequel la dispersion comprend un agent dispersant.

11. Produit cosmétique huile dans eau qui peut être obtenu par le procédé selon l'une quelconque des revendications 7 à 10.

12. Processus cosmétique pour le soin et/ou maquillage de matériaux kératiniques, comprenant l'application sur des matériaux kératiniques, en particulier sur la peau, du produit cosmétique huile dans eau tel que défini dans l'une quelconque des revendications 1 à 6 et 11.

13. Utilisation du produit cosmétique huile dans eau comme défini selon l'une quelconque des revendications 1 à 6 et 11 pour fournir aux matériaux kératiniques sur lesquelles il est appliqué une sensation de fraîcheur, un effet nourrissant, un effet couvrant et un effet longue durée.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008044901 A

- US 8728503 B

**Non-patent literature cited in the description**

- **KODA, Y.** Yuki Gainenzu - Kiso to Ouyou. Sankyo Publishing, 1984, 11-17